Europäisches Patentamt

(19) European Patent Office

Office européen des brevets

(11) EP 0 691 886 B1

(12) **FASCICULE DE BREVET EUROPEEN**

(45) Date de publication et mention
de la délivrance du brevet:
**28.04.1999 Bulletin 1999/17**

(21) Numéro de dépôt: **95908292.6**

(22) Date de dépôt: **01.02.1995**

(51) Int. Cl.$^6$: **B01J 13/16**, A61K 9/50,
A23L 1/22

(86) Numéro de dépôt international:
**PCT/FR95/00116**

(87) Numéro de publication internationale:
**WO 95/21018 (10.08.1995 Gazette 1995/34)**

(54) **MICROCAPSULES A PAROI DE POLYPHENOLS VEGETAUX RETICULES ET COMPOSITIONS EN CONTENANT**

MIKROKAPSELN MIT WÄNDEN AUS VERNETZTEN PFLANZLICHEN POLYPHENOLEN UND DIESE ENTHALTENDE ZUSAMMENSETZUNGEN

MICROCAPSULES WITH WALLS MADE OF CROSS-LINKED PLANT POLYPHENOLS, AND COMPOSITIONS CONTAINING SAME

(84) Etats contractants désignés:
**BE CH DE ES FR GB GR IT LI NL**

(30) Priorité: **02.02.1994 FR 9401146**

(43) Date de publication de la demande:
**17.01.1996 Bulletin 1996/03**

(73) Titulaire:
**CENTRE NATIONAL DE
LA RECHERCHE SCIENTIFIQUE (CNRS)
75794 Paris Cédex 16 (FR)**

(72) Inventeurs:
• **LEVY, Marie-Christine
F-51100 Reims (FR)**
• **ANDRY, Marie-Christine
F-51530 Dizy (FR)**

(74) Mandataire:
**Martin, Jean-Jacques et al
Cabinet REGIMBEAU
26, Avenue Kléber
75116 Paris (FR)**

(56) Documents cités:
• **CRITICAL REVIEWS IN FOOD SCIENCE AND NUTRITION, vol. 28,no. 4, 1889 pages 273-314, F. J. FRANCIS 'Food Colorants: Anthocyanins' cité dans la demande**
• **BIOCHEMICAL PHARMACOLOGY, vol. 44,no. 1, 7 Juillet 1992 pages 180-183, 'Picroliv, picroside-I and kutkoside from Picrorhisa kurrooa are scavangers of superoxide anions' cité dans la demande**
• **JOURNAL OF POLYMER SCIENCE, vol. XL, 1959 pages 399-406, W. M. EARECKSON 'Interfacial Polycondensation. X. Polyphenyl Esters' cité dans la demande**

**Description**

La présente invention concerne essentiellement l'application aux polyphénols végétaux de la réticulation interfaciale au moyen d'un agent réticulant pour former des microcapsules, les microcapsules ainsi réalisées, leurs procédés de fabrication ainsi que les compositions contenant les microcapsules ainsi obtenues telles que compositions cosmétiques, pharmaceutiques, alimentaires, diététiques.

Les polyphénols végétaux constituent un groupe important de substances naturelles dont les propriétés anti-radicalaires et anti-oxydantes sont bien connues (voir par exemple : "Polyphenolic Phenomena", A. SCALBERT, Editeur, INRA Editions, Paris, 1993). Ces composés, parmi lesquels notamment les flavonoïdes, tels que par exemple les oligomères procyanidoliques ou OPC, possèdent des propriétés biologiques intéressantes liées en particulier à leur activité anti-radicalaire. Par exemple, ils peuvent prévenir les effets nocifs des radicaux libres sur la peau et ainsi jouer un rôle de protection contre les radiations solaires, contre le vieillissement de la peau, un rôle anti-carcinogène. Ils peuvent aussi prévenir l'érythème et la couperose. En outre, ils possèdent des propriétés utilisables en thérapeutique, en particulier en dermatologie et pour applications sur les muqueuses, telles que des propriétés anti-inflammatoires, des propriétés vasculo-protectrices (traitement des ecchymoses, pétéchies, gingivorragies, épistaxis....), des propriétés anti-allergiques, anti-ulcéreuses, anti-bactériennes, anti-virales, anti-cancéreuses. Enfin, ajoutés à des aliments ou produits diététiques, ils peuvent tout a la fois assurer la conservation des préparations auxquels ils sont incorporés, par action anti-oxydante, et constituer un apport intéressant de substances anti-radicalaires, permettant la prévention des maladies dûes aux radicaux libres, telles que le cancer.

Ils ont ainsi des applications notamment dans les domaines cosmétique, pharmaceutique, alimentaire et diététique. Toutefois, il n'est souvent pas possible de les incorporer à certaines préparations, telles que par exemple des préparations à usage cosmétique ou dermatologique, en raison de la coloration foncée que ces substances relativement instables communiquent aux dites préparations.

De même les dérivés anthocyaniques, substances polyphénoliques colorées appartenant également au groupe des flavonoïdes (F.J. Francis, Crit. Rev. Food Sci. Nutri., 1989, 28, 273-314), présentent aussi une activité anti-radicalaire et sont doués de propriétés biologiques intéressantes, notamment sur la perméabilité et la résistance capillaire. Toutefois ils ne peuvent généralement pas être incorporées à certaines préparations telles que par exemple des préparations à usage cosmétique ou dermatologique en raison de leur fort pouvoir colorant.

Il est connu que l'on peut préparer des polymères de haut poids moléculaire par polycondensation interfaciale d'un diphénol synthétique, le bisphénol A, avec les chlorures de diacides ( W. M. Eareckson, J. Polymer Sci., 1959, 40, 399-406). Sur ce principe, S. Suzuki et al. (Chem. Pharm. Bull., 1968, 6, 1629-1631) ont obtenu des microcapsules en appliquant la réaction de polycondensation entre le bisphénol A et le chlorure de sébacoyle à une émulsion.

Toutefois, aucun document de la littérature antérieure ne décrit la préparation de microcapsules par réticulation interfaciale de polyphénols végétaux.

Dans le cadre de l'invention, il a été découvert de manière inattendue que si l'on réalisait une réticulation interfaciale de polyphénols végétaux, en particulier de flavonoïdes, au moyen d'un agent réticulant, de préférence un halogénure de diacide, en particulier un chlorure de diacide, on obtenait un produit, en particulier des microcapsules, particulièrement stable notamment en présence d'un milieu aqueux, tout en préservant l'activité initiale de ces polyphénols végétaux, en particulier une activité biologique, notamment anti-radicalaire, ce qui est particulièrement remarquable.

Ainsi, il a été observé de manière inattendue que l'acylation interfaciale de groupements phénoliques du polyphénol végétal forme des liaisons ester et donne des membranes de polyphénol réticulé, tout en laissant libres suffisamment de groupements phénoliques pour le maintien des propriétés des polyphénols végétaux, en particulier de leurs propriétés anti-radicalaires et anti-oxydantes.

Il a en outre été observé que ce produit, incorporé dans une composition, évite les risques d'instabilité de cette composition, notamment en ce qui concerne sa coloration, corrélatifs à la présence au sein de cette composition de polyphénols végétaux, en particulier de flavonoïdes, susceptibles de se dégrader.

Ainsi, la présente invention a principalement pour objet de résoudre le nouveau problème technique consistant en la fourniture d'une solution permettant de préparer un produit à partir de polyphénols végétaux, en particulier de flavonoïdes, qui, incorporé à une composition, n'en altère pas la stabilité, en particulier la stabilité de coloration.

La présente invention a également pour but principal de fournir une solution permettant d'empêcher la diffusion de polyphénols végétaux, en particulier de flavonoïdes, notamment à l'état dissous, dans l'ensemble de la composition dans laquelle ils sont incorporés.

Ainsi, la présente invention permet de prévenir toute altération, en particulier toute modification de coloration au cours du temps d'une composition contenant des polyphénols végétaux, en particulier des flavonoïdes.

La présente invention a encore pour but principal de fournir une solution permettant de préparer un produit qui ne tache pas la peau à partir de polyphénols végétaux, en particulier de flavonoïdes et en particulier de dérivés anthocyaniques, en permettant ainsi leur incorporation dans des préparations cosmétiques ou pharmaceutiques destinées à être appliquées sur la peau ou les muqueuses.

La présente invention a encore pour but principal de résoudre le nouveau problème technique consistant en la fourniture d'une solution permettant de préparer un produit de bonne conservation, en particulier de couleur stable à partir de polyphénols végétaux, en particulier de flavonoïdes, en rendant ainsi possible la préparation de compositions à usage cosmétique ou pharmaceutique, en particulier dermatologique, alimentaire ou diététique.

La présente invention a encore pour but principal de résoudre le nouveau problème technique consistant en la fourniture d'une solution permettant de préparer une forme stable de polyphénols végétaux, en particulier de flavonoïdes, tout en préservant l'activité initiale spécifique de ces polyphénols végétaux, en particulier de ces flavonoïdes.

La présente invention a encore pour but principal de résoudre le nouveau problème technique consistant en la fourniture d'une solution permettant de préparer un produit stable, en particulier des microcapsules, à partir de polyphénols végétaux, en particulier de flavonoïdes, préservant l'activité initiale de ces polyphénols végétaux, en particulier de ces flavonoïdes, tout en permettant éventuellement l'encapsulation d'une ou plusieurs substances actives à l'état de solution, de suspension ou d'émulsion, permettant ainsi d'accroître l'activité biologique de ce produit ou de ces microcapsules.

La présente invention a encore pour but de résoudre les nouveaux problèmes techniques énoncés ci-dessus avec l'utilisation de procédés de fabrication simples, utilisables à l'échelle industrielle, en particulier dans l'industrie cosmétique, pharmaceutique, alimentaire ou diététique. De préférence, cette solution doit permettre de préparer des microcapsules ayant une taille de particules réglable à volonté, en particulier dans une plage de dimension allant de moins d'un micromètre à plus d'un millimètre.

Ainsi, selon la présente invention, il a été découvert de manière parfaitement inattendue, que l'on pouvait obtenir des microcapsules en déclenchant une réaction de polycondensation entre un polyphénol végétal et un agent réticulant, de préférence un halogénure de diacide, en particulier un chlorure de diacide, à l'interface des phases d'une émulsion, en particulier de type "eau-dans-l'huile". Dans ce cas, on émulsionne tout d'abord la solution aqueuse du polyphénol végétal au sein d'une phase hydrophobe, puis on ajoute la solution d'agent réticulant à l'émulsion. On constate alors qu'il se forme à l'interface des gouttelettes aqueuses, par suite de l'établissement de liaisons ester entre l'agent réticulant et des fonctions phénol du polyphénol végétal, des membranes constituées de molécules réticulées du polyphénol végétal. Après réaction, ces membranes forment donc des microcapsules qui peuvent être facilement séparées du milieu réactionnel et soumises à des lavages qui éliminent le polyphénol végétal non lié à la membrane.

D'autre part, ces microcapsules sont suffisamment stables pour subir une lyophilisation sans aucune destruction de leur structure et reprennent une forme sphérique après réhydratation, ce qui constitue encore un avantage technique déterminant de l'invention.

Il a été également découvert que l'on pouvait obtenir des microcapsules en déclenchant la réaction de polycondensation au sein d'une émulsion de type "huile-dans-l'eau". Dans ce cas, on émulsionne une phase hydrophobe contenant un agent réticulant, de préférence un halogénure de diacide, en particulier un chlorure de diacide, au sein d'une phase aqueuse contenant le polyphénol végétal et utilisée comme phase dispersante. On laisse la réaction se développer à l'interface et on maintient l'agitation pendant un temps convenable. On constate qu'il se forme une membrane autour des gouttelettes hydrophobes dispersées, donnant ainsi des microcapsules à contenu hydrophobe.

Ainsi, selon un premier aspect, la présente invention couvre des microcapsules caractérisées en ce qu'elles comprennent une paroi formée d'un ou plusieurs polyphénols végétaux réticulés, en particulier au moyen d'une réticulation interfaciale entre le ou les polyphénols végétaux et un agent réticulant, de préférence un halogénure de diacide, en particulier un chlorure de diacide.

Selon un mode de réalisation avantageux, ces microcapsules sont caractérisées en ce qu'elles comprennent une protéine ou un polysaccharide, ou un polyalkylèneglycol, ou un mélange quelconque de ces substances. Avantageusement, la paroi des microcapsules peut également comprendre une protéine et/ou un polysaccharide et/ou un polyalkylèneglycol co-réticulé avec le polyphénol végétal précité.

Selon un mode de réalisation particulièrement avantageux, la protéine précitée peut être douée d'une activité biologique spécifique, telle qu'une activité enzymatique, comme par exemple la catalase, la superoxyde dismutase, ou la glutathion peroxydase, et dans ce cas cette activité peut s'ajouter utilement à l'activité propre du ou des polyphénols végétaux, en particulier du ou des flavonoïdes.

Selon un autre mode de réalisation avantageux de l'invention, les microcapsules précitées peuvent être préparées à partir d'un seul polyphénol végétal ou de mélanges d'origine naturelle ou non contenant des polyphénols végétaux, tels que par exemple les jus de fruits ou les extraits de plantes ou de parties de plantes.

Selon un autre mode de réalisation particulièrement avantageux de l'invention, les polyphénols végétaux précités peuvent être des polyphénols végétaux monocycliques ou polycycliques, tels que les flavonoïdes, les isoflavonoïdes, les néoflavonoïdes, les tanins galliques et les tanins ellagiques, le catéchol et ses dérivés tels que la DL-3,4-dihydroxyphénylalanine ou DL-DOPA, ou les catécholamines telles que la 3-hydroxytyramine ou dopamine, ou le phloroglucinol, ou les acides phénols tels que l'acide caféique, l'acide dihydrocaféique, l'acide protocatéchique, l'acide chlorogénique l'acide isochlorogénique, l'acide gentisique, l'acide homogentisique, l'acide gallique, l'acide hexahydroxydiphénique, l'acide ellagique, l'acide rosmarinique, l'acide lithospermique, ou les dérivés des acides phénols, en particulier leurs esters ou leurs hétérosides, ou la curcumine, ou les coumarines polyhydroxylées, ou les lignanes ou les néolignanes polyhydroxylés, ou un mélange contenant un ou plusieurs polyphénols végétaux ou leurs dérivés, comme la silymarine. En particulier, tous les polyphénols précités peuvent être utilisés sous forme de préparations obtenues à partir de plantes ou de parties de plantes, telles que des extraits, des teintures, des jus de fruits, des vins.

Les polyphénols végétaux particulièrement avantageux dans le cadre de l'invention sont ceux qui sont extraits notamment des plantes appartenant aux genres suivants : Gingko, Lespedeza, Passiflora, Silybum, Citrus, Hamamelis, Thymus, Chamaemelum, Achillea, Equisetum, Sophora, Fagopyrum, Eucalyptus, Sambucus, Betula, Vitis, Pinus, Crataegus, Quercus, Ratanhia, Lythrum, Acacia, Cupressus, Vaccinium, Ribes, Centaurea, Rosa, Hibiscus, Malva, Podophyllum, Schizandra, Gaïacum, Arctostaphylos, Cynara, Rosmarinus, Orthosiphon, Solidago, Lithospermum, Curcuma, Aesculus, Melilotus, Ammi, Hieracium, Angelica, Asperula.

Selon une variante de réalisation avantageuse, les polyphénols végétaux précités sont des flavonoïdes choisis parmi le groupe consistant d'une flavone, telle que l'apigénol, le lutéolol, un flavonol comme la quercétine, le kaempférol, ou un hétéroside de flavone ou de flavonol, comme la rutine et ses dérivés, une flavanone comme la flavanone, la naringénine, l'hespérétine, ou un hétéroside de flavanone comme la naringine, l'hespéridine, la diosmine, ou un dérivé de flavanone comme le diosmoside, ou un biflavonoïde, ou un dimère de flavone ou de flavanone, comme l'amentoflavone, ou une chalcone telle que l'isoliquirtigénine ou l'hespéridine méthylchalcone, un flavanonol tel que le taxifoliol ou une substance dérivée de taxifoliol telle que la silybine, la silychristine, la silydianine, un flavan-3-ol tel que la (+) catéchine, la (-) épicatéchine, un polymère formé d'unités de structure de base flavan-3-ol, généralement désigné sous le nom de "proanthocyanidine" ou sous l'expression "tanin condensé", en particulier un oligomère comprenant de 2 à 8 de ces unités, appelé généralement "oligomère procyanidolique" (OPC), un anthocyanoside comme le malvoside ou un mélange contenant un ou plusieurs flavonoïdes, en particulier sous la forme d'extraits de fruits ou d'extraits de plantes ou de parties de plantes.

En particulier, le mélange de flavonoïdes précité est de préférence choisi dans le groupe constitué par des mélanges de citroflavonoïdes extraits de divers Citrus (Rutacées), un mélange de flavonoïdes extrait de Silybum marianum (Composées) ou la silymarine, les extraits de Gingko biloba (Ginkgoacées), les extraits riches en anthocyanosides de myrtille, de fruits de cassis, de peaux de raisin, de feuille de vigne rouge, les jus de fruits tels que les lus de raisin, de cassis, tels quels ou concentrés ou desséchés notamment par nébulisation ou lyophilisation, les vins rouges, tels quels ou concentrés ou desséchés, ou leurs divers mélanges.

Selon un autre mode de réalisation avantageux de l'invention, la protéine précitée peut être choisie parmi le groupe consistant des albumines comme la sérumalbumine, l'ovalbumine, l'alpha-lactalbumine, les globulines, le fibrinogène, la caséine, les protéines végétales telles que les protéines du soja, les glutélines qui de préférence auront été dégradées, les scléroprotéines solubilisées, le collagène, l'atélocollagène, la gélatine, les hydrolysats de gélatine, les peptones, l'hémoglobine, les enzymes telles que la catalase, la superoxyde dismutase, la glutathion peroxydase, des mélanges contenant des protéines hydrophiles, tels que le lait entier ou écrémé totalement ou partiellement, le lait en poudre, le lait condensé, les protéines du lactosérum, la farine de soja, les mélanges d'atélocollagène et de glycosaminoglycanes.

Selon un autre mode de réalisation avantageux de l'invention, le polysaccharide précité peut être choisi parmi le groupe consistant des dextrans, de l'acide alginique et ses sels hydrosolubles, en particulier l'alginate de sodium, les gommes végétales, les carraghénanes, les pectines, les dérivés solubles d'amidon, les dérivés solubles de cellulose, les glycosaminoglycanes.

Selon un autre mode de réalisation avantageux de l'invention, le polyalkylèneglycol peut être choisi parmi le groupe consistant des polyéthylèneglycols et des polypropylèneglycols.

Selon encore un autre mode de réalisation avantageux de l'invention, les microcapsules précitées sont préparées par réticulation interfaciale à partir d'une émulsion dont la phase aqueuse contient de 1 % à 40 %, de préférence entre 1 et 20 % en poids de polyphénols végétaux par rapport au poids total de la phase aqueuse. Lorsque une protéine précitée et/ou un polysaccharide précité et/ou un polyalkylèneglycol précité est présent, la concentration totale dans la phase aqueuse de cette, ou de ces, substance(s) est comprise avantageusement entre 0,1 et 30 % en poids, de préférence entre 1 et 10 % en poids, par rapport au poids total de la phase aqueuse.

Selon encore un autre mode de réalisation avantageux de l'invention, les microcapsules précitées sont préparées par réticulation interfaciale à partir d'une émulsion dont la phase dispersée à encapsuler renferme une ou plusieurs substances actives hydrosolubles, liposolubles ou insolubles, incorporées à l'état de solution, de suspension ou d'émulsion, en particulier une substance minérale réflectrice des radiations solaires, de préférence insoluble, une huile végétale, ou une solution huileuse contenant une substance active lipophile telle qu'un filtre solaire liposoluble. Ainsi, les microcapsules obtenues contiennent lesdites substances hydrosolubles, liposolubles ou insolubles.

Selon un mode de réalisation particulièrement avantageux, les substances actives incorporées dans les microcapsules selon l'invention peuvent être choisies parmi le groupe consistant d'une substance minérale réflectrice des radiations solaires, telle qu'un oxyde de fer, l'oxyde de titane, l'oxyde de zinc, le talc, le kaolin, une huile végétale telle qu'une huile de germes de céréales ou une huile de foie de poisson désodorisée, ou une solution huileuse d'une substance liposoluble telle que la vitamine A, la vitamine D2, la vitamine E ou tocophérol, un acide gras essentiel tel que l'acide linoléique, l'acide linolénique, l'acide arachidonique, une céramide, un dérivé liposoluble d'acide ascorbique tel que le palmitate d'ascorbyle, ou un filtre solaire liposoluble tel qu'un ester cinnamique, un ester paraaminobenzïoque, un ester salicylique, une benzophénone, le benzylidène camphre et ses dérivés, un dérivé du dibenzoylméthane, un benzimidazole, ou une substance photoactive telle que le bergaptène ou tout autre dérivé du psoralène, ou encore un mélange contenant plusieurs substances actives.

Selon un deuxième aspect, la présente invention couvre également un procédé de fabrication des microcapsules telles que précédemment définies, caractérisé en ce qu'on réalise une réticulation interfaciale d'une émulsion de type eau-dans-l'huile, comprenant les étapes essentielles suivantes :

a) on prépare une phase aqueuse contenant le polyphénol végétal ou le mélange de polyphénols végétaux à réticuler,

b) on prépare une phase hydrophobe, contenant éventuellement un ou plusieurs agents tensio-actifs,

c) on émulsionne ladite phase aqueuse dans la phase hydrophobe précitée, de sorte que la phase hydrophobe constitue la phase continue dans laquelle la phase aqueuse forme la phase dispersée,

d) on ajoute à l'émulsion ainsi obtenue l'agent réticulant dissous dans un liquide miscible à la phase hydrophobe, sous agitation, pour réaliser une réticulation interfaciale de l'agent réticulant et du ou des polyphénols végétaux contenus dans la phase aqueuse,

e) on maintient l'agitation pendant un temps de réaction convenable pour obtenir une réticulation suffisante conduisant à la formation de microcapsules dont la paroi comprend le ou les polyphénols végétaux réticulés par l'agent réticulant,

f) on recueille les microcapsules ainsi formées, par tout moyen approprié.

Selon un troisième aspect, la présente invention couvre également un procédé de fabrication des microcapsules précédemment définies, caractérisé en ce qu'on réalise une réticulation interfaciale d'une émulsion de type huile-dans-eau, comprenant les étapes essentielles suivantes :

a) on prépare une phase hydrophobe dans laquelle on dissout l'agent réticulant,

b) on prépare une phase aqueuse contenant le polyphénol végétal ou le mélange de polyphénols végétaux à réticuler, et éventuellement un ou plusieurs agents tensioactifs,

c) on émulsionne la phase hydrophobe dans la phase aqueuse précitée, de sorte que la phase aqueuse constitue la phase continue dans laquelle la phase hydrophobe forme la phase dispersée,

d) on maintient l'ensemble sous agitation pendant un temps de réaction convenable pour obtenir une réticulation suffisante conduisant à la formation de microcapsules dont la paroi comprend le ou les polyphénols végétaux réticulés par l'agent réticulant,

e) on recueille les microcapsules ainsi formées, par tout moyen approprié.

L'étape d'émulsification, de l'un ou l'autre des procédés des deux aspects précédents, est réalisée en utilisant l'une des techniques bien connues de l'homme du métier, notamment en jouant sur les proportions respectives de la phase aqueuse et de la phase hydrophobe, et/ou en utilisant un ou plusieurs agents tensio-actifs appropriés dispersés dans la phase hydrophobe et/ou dans la phase aqueuse.

Pour obtenir une émulsion de type eau-dans-huile, on utilisera de préférence un ou plusieurs agents tensio-actifs choisis en particulier parmi les esters de sorbitan, tels que le Span 85®, les esters gras de glycérol, tels que le monooléate de glycérol, les esters gras de glycols, tels que le stéarate d'éthylèneglycol.

Pour obtenir une émulsion de type huile-dans-eau, on utilisera de préférence un ou plusieurs agents tensio-actifs choisis par exemple parmi les esters gras de sorbitan polyoxyéthylénés, ou Tween®, en particulier le Tween 20®. Il est à noter toutefois que, dans le cadre des procédés selon l'invention, la présence d'un agent tensio-actif n'est pas critique.

Cette étape d'émulsification est réalisée sous agitation par tout moyen approprié, en particulier par agitation mécanique ou au moyen d'ultra-sons ou encore au moyen d'un homogénéiseur haute pression, par exemple un homogénéiseur fonctionnant jusqu'à 700 bars, disponible dans le commerce. L'utilisation d'un homogénéiseur haute pression est particulièrement avantageuse lorsque l'on souhaite obtenir des gouttelettes particulièrement fines.

En général, il est nécessaire de réaliser des lavages des microcapsules obtenues après réticulation, afin d'éliminer l'excès d'agent réticulant ainsi que le ou les polyphénols végétaux n'ayant pas réagi ou non encapsulés dans les microcapsules. Pour effectuer cette opération, les microcapsules sont séparées du milieu réactionnel par centrifugation ou décantation ou tout autre moyen approprié. Dans le cas du procédé comprenant l'émulsification eau-dans-huile, les microcapsules sont lavées par remise en suspension successivement dans un liquide hydrophobe tel que l'un des liquides utilisés pour constituer la phase hydrophobe de l'émulsion initiale, puis dans un alcool tel que l'éthanol ou le méthanol, purs ou dilués d'eau, puis dans l'eau. Dans le cas du procédé comprenant l'émulsification huile-dans-eau, les microcapsules sont lavées à l'eau ou dans une solution aqueuse appropriée.

Selon une variante de réalisation de l'un ou l'autre des procédés des aspects précédents, on ajoute à la phase aqueuse lors de sa préparation une protéine et/ou un polysaccharide et/ou un polyalkylèneglycol, la paroi des microcapsules formées par réaction de réticulation pouvant comprendre le ou les polyphénols végétaux co-réticulés avec ladite protéine et/ou ledit polysaccharide et/ou ledit polyalkylèneglycol, par l'agent réticulant.

L'incorporation de polyalkylèneglycols, en particulier d'un polyéthylèneglycol, à la phase aqueuse des microcapsules permet d'augmenter l'hydrophilie de ces microcapsules, surtout dans le cas où le produit polyphénolique utilisé pour la préparation des microcapsules présente un caractère relativement hydrophobe. Cette hydrophilie peut être réglée en augmentant la proportion de polyalkylèneglycol incorporé. Ainsi, ces microcapsules, placées en milieux aqueux, reprennent rapidement une forme sphérique et donnent des sédiments très faciles à disperser. De plus, les échanges entre l'intérieur des microcapsules et l'extérieur seront facilités, ce qui pourra permettre en particulier à des constituants du milieu extérieur de venir plus facilement au contact de groupements fonctionnels portés par la face interne de la membrane, augmentant ainsi l'activité des microcapsules. Par ailleurs, la présence d'un polyalkylèneglycol, en particulier d'un polyéthylèneglycol, dans les microcapsules, peut améliorer la rétention de molécules hydrosolubles encapsulées.

Selon un mode de réalisation avantageux de ces procédés, l'agent réticulant comprend un halogénure de diacide, en particulier un chlorure de diacide, de préférence choisi parmi le groupe consistant d'un chlorure de diacide aliphatique ou aromatique, tel que le chlorure de sébacoyle, le chlorure de succinyle, le chlorure d'adipoyle, le chlorure de téréphtaloyle, le chlorure de glutaryle. La concentration en halogénure de diacide sera de préférence comprise entre 0,2 % et 10 % en poids du poids total du milieu réactionnel.

Par ailleurs, le temps de réaction est naturellement variable en fonction des réactifs utilisés et notamment de la nature de l'halogénure, en particulier du chlorure de diacide. Généralement, le temps de réaction sera compris entre 5 min et 2 h et avantageusement sera compris entre 15 et 60 min.

D'autre part, il est avantageux que le pH de la réaction soit compris entre 8 et 14, et encore mieux entre 9 et 12. Pour assurer ce pH réactionnel, on peut utiliser des solutions tampons ou des solutions d'un agent alcalin, tel que la soude ou la potasse.

Comme substances utilisées pour constituer la phase hydrophobe, on pourra utiliser des substances liquides bien connues de l'homme de l'art.

Les substances liquides hydrophobes actuellement préférées sont choisies parmi le groupe consistant des hydrocarbures halogénés ou non, tels que le cyclohexane, le chloroforme ou le dichlorométhane, des esters d'acides gras, tels que le myristate d'isopropyle ou l'oléate d'éthyle, des mélanges d'esters d'acides gras disponibles dans le commerce, tels que par exemple le produit Dragoxat®, commercialisé par la firme DRAGOCO, des huiles végétales, telles que l'huile d'olive, l'huile d'amande douce ou l'huile d'arachide, des huiles minérales, telles qu'une huile de paraffine, et tout mélange de ces substances liquides hydrophobes.

Suivant une variante selon l'un quelconque des procédés précités, on incorpore à la phase devant être dispersée, constituant la phase liquide à encapsuler, une ou plusieurs substances actives, à l'état de solution, de suspension ou d'émulsion, en particulier une substance minérale réflectrice des radiations solaires, de préférence insoluble, une huile ou une solution huileuse de substance liposoluble, telle qu'un filtre solaire liposoluble.

Ainsi, le procédé selon l'invention permet de régler à volonté la taille des microparticules, en particulier dans une plage de dimension allant de moins d'un micromètre à plus d'un millimètre. Généralement, le diamètre des microcapsules selon l'invention se situe dans l'intervalle compris entre 0,1 $\mu$m (micromètre) et 3 mm.

Selon encore un autre mode de réalisation avantageux de l'invention, les microcapsules à paroi de polyphénols végétaux réticulés selon la présente invention peuvent être utilisées brutes de fabrication, dites fraîches. Dans ce cas, elles contiennent de la phase dispersée, aqueuse ou hydrophobe, ce qui facilite leur incorporation à des véhicules respectivement hydrophiles ou hydrophobes.

Egalement, selon un mode de réalisation avantageux de l'invention, les microcapsules précitées peuvent se présenter sous forme d'une suspension aqueuse dont on peut ajuster la concentration, et qui est directement incorporable aux formulations comportant un véhicule hydrophile.

Selon un autre mode de réalisation avantageux de l'invention, les microcapsules à paroi de polyphénols végétaux réticulés selon la présente invention peuvent être sous forme desséchée, notamment par lyophilisation, ce qui constitue un moyen aisé et fiable de stockage.

Ainsi, selon un mode de réalisation avantageux, les microcapsules précitées se présentent sous forme d'une poudre lyophilisée. Dans le cadre de l'invention, les microcapsules à paroi de polyphénols réticulés reprennent aisément une forme sphérique après réhydratation tout en conservant leur activité initiale.

Selon un quatrième aspect, la présente invention couvre encore des compositions, en particulier des compositions cosmétiques ou pharmaceutiques, notamment dermatologiques, des compositions diététiques, ou des compositions alimentaires, caractérisées en ce qu'elles comprennent des microcapsules à paroi de polyphénols végétaux réticulés telles que précédemment définies.

Selon un mode de réalisation avantageux, la concentration en microcapsules de polyphénols végétaux selon l'invention sera comprise entre 0,01 et 10 % en poids du poids total de la composition finale, encore mieux entre 0,1 et 5 % en poids de la composition finale.

Comme il a été dit précédemment, les microcapsules de l'invention à paroi de polyphénols végétaux réticulés conservent l'activité initiale des polyphénols végétaux. En conséquence, elles sont particulièrement utiles pour agir comme piégeurs de radicaux libres grâce à leur activité anti-radicalaire. De ce fait, ces microcapsules sont avantageusement utilisées dans des compositions cosmétiques ou pharmaceutiques, notamment dermatologiques, destinées à prévenir le vieillissement cutané, notamment le vieillissement actinique.

Ainsi également, et selon un cinquième aspect, la présente invention couvre encore une méthode de traitement cosmétique ou pharmaceutique, notamment dermatologique, d'un être humain pour la prévention du vieillissement cutané, notamment du vieillissement actinique dû généralement aux radicaux libres, caractérisée en ce qu'on applique une quantité efficace de microcapsules à paroi de polyphénols végétaux réticulés selon l'invention, éventuellement incluses dans un excipient, véhicule ou support cosmétiquement ou pharmaceutiquement acceptable, sur les zones de la peau ou des cheveux sensibles à l'action des radicaux libres, notamment les radicaux libres résultant d'une exposition actinique. Lors de ce traitement, la concentration en microcapsules sera habituellement comprise entre 0,01 et 10 % en poids, et de préférence entre 0,1 et 5 % en poids de la composition contenant les microcapsules.

Naturellement, ces compositions peuvent se présenter sous diverses formes cosmétiquement, pharmaceutiquement, alimentairement ou diététiquement acceptables. Ces formes sont bien connues de l'homme de l'art. On citera à titre d'exemples non limitatifs des crèmes, des pommades, des lotions, notamment des lotions capillaires, des gels, des laits, des suspensions, des poudres, des gélules.

Enfin, selon un sixième aspect, la présente invention couvre encore un procédé de préparation d'une composition incorporant un ou plusieurs polyphénols végétaux, caractérisé en ce qu'en vue de prévenir toute altération, notamment toute modification de coloration de la composition au cours du temps, tout en conservant l'activité des polyphénols végétaux, notamment l'activité anti-radicalaire, et/ou antioxydante et/ou biologique, ces derniers sont incorporés dans ladite composition sous forme de microcapsules telles que définies précédemment ou obtenues par la mise en oeuvre de l'un des procédés décrits ci-dessus.

Il est à noter que dans le cadre de l'un ou l'autre des aspects précédents, les diverses variantes de réalisation n'ont pas été répétées mais il est bien clair que l'invention couvre pour chacun des aspects, et de manière indépendante, chacune et toutes les variantes de réalisation qui ont pu être décrites précédemment ou dans la suite de la description pour l'un ou l'autre des aspects de l'invention.

D'autres buts, caractéristiques et avantages de l'invention apparaîtront clairement à la lumière de la description explicative qui va suivre faite en référence à divers exemples de réalisation de l'invention donnés simplement à titre d'illustration et qui ne sauraient donc en aucune façon limiter la portée de l'invention. Dans les exemples, tous les pourcentages sont donnés en poids, sauf indication contraire.

Exemple 1

Fabrication de microcapsules constituées d'oligomères procyanidoliques d'écorce de pin (OPC EP ) réticulés par le chlorure de téréphtaloyle (CT)

a) Préparation de la phase aqueuse

Dans 3 ml d'un tampon carbonate pH 11 on dissout 300 mg d'OPC EP (SARPAP).

b) Emulsification

On émulsionne 3 ml de cette solution dans 15 ml de cyclohexane additionné de 5 % de Span 85<sup>®</sup>, par agitation à 3000 rpm.

c) Addition de l'agent réticulant

Après 5 min, on ajoute à l'émulsion 20 ml d'une solution à 5 % (p/v) de CT (Janssen Chimica) dans un mélange de chloroforme: cyclohexane 1:4 (v/v) et l'agitation est maintenue pendant 30 min.

d) Lavages

Les microcapsules sont séparées par centrifugation et lavées par remise en suspension successivement dans le cyclohexane, dans l'éthanol à 95 % additionné de 2 % de Tween 20<sup>®</sup>, dans l'alcool à 95 % et finalement dans l'eau distillée.

On obtient un sédiment beige de microcapsules. L'examen en microscopie optique montre de belles microcapsules rondes, transparentes, très indépendantes, de taille 5 à 20 $\mu$m. Les microcapsules sont intactes après lyophilisation. L'examen en microscopie électronique à balayage montre des particules bien individualisées avec une membrane continue.

Stabilité : A l'état de suspension aqueuse, les microparticules peuvent être conservées au moins 7 mois à +4°C, au moins 3 mois 1/2 à 20°C, et au moins 3 mois 1/2 à + 45°C: les microcapsules sont intactes, leur couleur n'est pas modifiée, et le surnageant est incolore.

Activité anti-radicalaire des microcapsules lyophilisées :

La mesure de l'activité anti-radicalaire a été effectuée conformément au test d'activité décrit à l'exemple 37.

Le résultat de ce test est exprimé en pourcentage de piégeage des radicaux libres par rapport à un échantillon témoin ne contenant pas de piégeur de radicaux libres. Par ailleurs, on a également mesuré la conservation de l'activité antiradicalaire, donc de piégeage des radicaux libres, en effectuant une comparaison avec des flavonoïdes non réticulés, ici des oligomères procyanidoliques de pépins de raisin (OPC PR).

Les résultats figurent au tableau II en fin de description. On rappelle ici que l'activité anti-radicalaire des microcapsules selon l'invention de cet exemple est de 81 ± 4 % ce qui est essentiellement similaire à l'activité anti-radicalaire des OPC PR de 91 ± 2 %, ce qui démontre la conservation de l'activité initiale des flavonoïdes malgré la réticulation.

Exemple 2

Fabrication de microcapsules constituées d'oligomères procyanidoliques d'écorce de pin (OPC EP) réticulés par le chlorure de téréphtaloyle (CT)

a) Préparation de la phase aqueuse

Dans 3 ml d'un tampon carbonate pH 9,8 on dissout 300 mg d'OPC EP (SARPAP).

b) Emulsification

On émulsionne 3 ml de cette solution dans 15 ml de cyclohexane additionné de 5 % de Span 85<sup>®</sup>, par agitation à 3 000 rpm.

c) Addition de l'agent réticulant

Après 5 min, on ajoute à l'émulsion 20 ml d'une solution à 2,5 % (p/v) de CT (Janssen Chimica) dans un mélange de chloroforme: cyclohexane 1:4 (v/v) et l'agitation est maintenue pendant 30 min.

On obtient un sédiment de couleur chamois formé de microcapsules transparentes de diamètre moyen 30 $\mu$m. Les microcapsules sont intactes après un an de conservation à l'état de suspension aqueuse à 20°C. leur couleur n'a pas changé et le surnageant est parfaitement incolore.

Exemple 3

Fabrication de microcapsules constituées d'oligomères procyanidoliques d'écorce de pin (OPC EP) et de sérumalbumine bovine (BSA) co-réticulés par le chlorure de téréphtaloyle (CT).

Le protocole décrit à l'exemple 1 est appliqué en utilisant comme phase aqueuse une solution renfermant 10 % d'OPC EP et 5% de BSA (Fraction V, Sigma) dans le tampon pH 11.

On obtient un sédiment beige formé de belles microcapsules indépendantes, de diamètre 5-10 $\mu$m.

Stabilité : A l'état de suspension aqueuse, les microparticules peuvent être conservées au moins 7 mois à +4°C, au moins 1 mois à 20°C, et au moins 3 semaines à +45°C : les microcapsules sont intactes, leur couleur n'est pas modifiée, et le surnageant est incolore.

Résultats de la détermination de l'activité anti-radicalaire sur microcapsules lyophilisées : le piégeage est de 51±1 %. (voir tableau II)

Exemple 4

Fabrication de microcapsules constituées d'oligomères procyanidoliques d'écorce de pin (OPC EP) et d'ovalbumine réticulés par le chlorure de téréphtaloyle (CT).

Le protocole décrit à l'exemple 1 est appliqué en utilisant comme phase aqueuse une solution renfermant 10 % d'OPC EP et 5 % d'ovalbumine (Sigma) dans le tampon carbonate pH 11.

On obtient un sédiment formé de microcapsules de diamètre 10-80 $\mu$m.

Exemple 5

Fabrication de microcapsules constituées d'oligomères procyanidoliques de pépins de raisins (OPC PR ) réticulés par le chlorure de téréphtaloyle (CT).

Le protocole décrit à l'exemple 1 est appliqué en utilisant comme phase aqueuse une solution d'oligomères procyanidoliques de pépins de raisins (OPC PR, SARPAP) à 10 % dans le tampon pH 11.

On obtient un sédiment chamois formé de belles microcapsules sphériques, transparentes de diamètre 5-25 $\mu$m. L'examen en microscope électronique à balayage montre des particules indépendantes à membrane continue. Stabilité : A l'état de suspension aqueuse, les microparticules peuvent être conservées au moins 6 mois à +4°C, au moins 1 mois à 20°C, et au moins 3 semaines à +45°C : les microcapsules sont intactes, leur couleur n'est pas modifiée, et le surnageant est incolore.

Résultats de la détermination de l'activité anti-radicalaire sur microcapsules lyophilisées : le piégeage est de 71±2 %. (Voir tableau II)

Exemple 6

Fabrication de microcapsules constituées d'oligomères procyanidoliques de pépins de raisins (OPC PR) et de protéines du lactosérum co-réticulés par le chlorure de téréphtaloyle (CT).

Le protocole décrit à l'exemple 1 est appliqué en utilisant comme phase aqueuse une solution renfermant 10 % d'oligomères procyanidoliques de pépins de raisins (OPC PR, SARPAP) et 5 % d'un concentré de protéines du lactosérum (Prosobel S65E, Bel Industries), dans le tampon pH 11.

On obtient un volumineux sédiment marron clair de microcapsules sphériques de diamètre 5-20 $\mu$m. L'examen en microscopie électronique à balayage montre des particules indépendantes à membrane continue.

Stabilité : A l'état de suspension aqueuse, les microparticules peuvent être conservées au moins 7 mois à +4°C, au moins 1 mois à 20°C, et au moins 3 semaines à +45°C : les microcapsules sont intactes, leur couleur n'est pas modifiée, et le surnageant est incolore.

Activité anti-radicalaire des microcapsules lyophilisées : le piégeage est de 83±1 %. (Voir tableau II)

Exemple 7

Fabrication de microcapsules constituées d'oligomères procyanidoliques de pépins de raisins(OPC PR) réticulés par le chlorure de sébacoyle (CS)

a) Préparation de la phase aqueuse

Dans 3 ml de soude 2 M, on dissout 300 mg d'OPC PR (SARPAP).

b) Emulsification

On émulsionne 3 ml de cette solution dans 15 ml de cyclohexane additionné de 5 % de Span 85$^®$, par agitation à 3 000 rpm.

c) Addition de l'agent réticulant

Après 5 min, on ajoute à l'émulsion 20 ml d'une solution à 5 % (v/v) de CS (SIGMA) dans un mélange de chloroforme: cyclohexane 1:4 (v/v) et l'agitation est maintenue pendant 30 min.

d) Lavages

Ils sont effectués comme décrit à l'exemple 1.
On obtient des microcapsules de taille 5-15 $\mu$m. L'examen en microscopie électronique à balayage montre des particules indépendantes à membrane continue.

Exemple 8

Fabrication de microcapsules constituées d'oligomères procyanidoliques de pépins de raisins(OPC PR) et de protéines du lactosérum co-réticulés par le chlorure de sébacoyle (CS).

Le protocole décrit à l'exemple 7 est reproduit en utilisant comme phase aqueuse une solution renfermant 10 % d'OPC PR et 5 % d'un concentré de protéines du lactosérum (Prosobel S65E, Bel Industries) dans la soude 2 M.
On obtient un volumineux sédiment formé de belles microcapsules de diamètre 10-20 $\mu$m.

Exemple 9

Fabrication de microcapsules constituées de catéchine réticulée par le chlorure de téréphtaloyle (CT).

Le protocole décrit à l'exemple 1 est appliqué en utilisant comme phase aqueuse une solution à 10 % de catéchine ((+)catechin,SIGMA) dans la soude 2 M.
On obtient un sédiment ocre de belles microcapsules de diamètre 5-15 $\mu$m.
Stabilité : A l'état de suspension aqueuse, les microparticules peuvent être conservées au moins 1 mois à 20°C, et au moins 3 semaines à + 45°C : les microcapsules sont intactes, leur couleur n'est pas modifiée, et le surnageant est incolore.
Activité anti-radicalaire des microcapsules lyophilisées: le piégeage est de 79±3 %. (Voir tableau II)

Exemple 10

Fabrication de microcapsules constituées de catéchine et d'ovalbumine co-réticulées par le chlorure de téréphtaloyle (CT).

Le protocole décrit à l'exemple 1 est appliqué en utilisant comme phase aqueuse une solution renfermant 10 % de catéchine et 2 % d'ovalbumine (SIGMA) dans la soude 2 M.
On obtient un sédiment de microcapsules indépendantes de diamètre 10-20 $\mu$m.

Exemple 11

Fabrication de microcapsules constituées de catéchine et de protéines du lactosérum co-réticulées par le chlorure de téréphtaloyle.

Le protocole décrit à l'exemple 1 est appliqué en utilisant comme phase aqueuse une solution renfermant 10 % de catéchine et 3 % d'un concentré de protéines du lactosérum (Prosobel S65E, Bel Industries), dans la soude 2 M.
On obtient un volumineux sédiment jaune formé de belles microcapsules de diamètre 5-20 μm.
Stabilité : A l'état de suspension aqueuse, les microparticules peuvent être conservées au moins 1 mois à 20°C, et au moins 3 semaines à + 45°C : les microcapsules sont intactes, leur couleur n'est pas modifiée, et le surnageant est incolore.
Résultats de la détermination de l'activité anti-radicalaire sur microcapsules lyophilisées : le piégeage est de 64±5 %.

Exemple 12

Fabrication de microcapsules constituées de catéchine réticulée par le chlorure de sébacoyle (CS).

Le protocole décrit à l'exemple 7 est reproduit en utilisant comme phase aqueuse une solution renfermant 10 % de catéchine dans la soude 2 M.
On obtient des microcapsules de diamètre 5-25 μm.

Exemple 13

Fabrication de microcapsules constituées de catéchine et d'ovalbumine co-réticulées par le chlorure de sébacoyle (CS).

Le protocole décrit à l'exemple 7 est appliqué en utilisant comme phase aqueuse une solution renfermant 10 % de catéchine et 2 % d'ovalbumine dans la soude 2 M.
On obtient un volumineux sédiment formé de belles microcapsules de diamètre 5-20 μm.

Exemple 14

Fabrication de microcapsules constituées de catéchine et de protéines du lait co-réticulées par le chlorure de sébacoyle (CS).

Préparation de la phase aqueuse :

On mélange 4 ml de lait (Viva, CANDIA) à 2 ml de soude 6 M. On dissout dans cette solution de la catéchine à la concentration de 10 %.
On utilise 3 ml de la solution obtenue pour l'émulsionner dans les conditions décrites à l'exemple 7. La réticulation et les lavages sont ensuite effectués comme décrit à l'exemple 7.
On obtient un volumineux sédiment formé de belles microcapsules de diamètre 5-25 μm.

Exemple 15

Fabrication de microcapsules constituées d'oligomères procyanidoliques de pépins de raisins (OPC PR) réticulés par le chlorure de téréphtaloyle (CT).

Le protocole décrit à l'exemple 1 est appliqué en utilisant comme phase aqueuse une solution d'oligomères procyanidoliques de pépins de raisins (Leucocianidine, INDENA) à 10 % dans le tampon pH 11, et une vitesse d'agitation de 5 000 rpm. Les mirocapsules sont lavées successivement dans le cyclohexane, dans l'éthanol à 95 % additionné de 2 % de Tween 20[®], dans le méthanol et finalement dans l'eau distillée.
On obtient un sédiment de couleur chamois formé de belles microcapsules sphériques, transparentes. Le diamètre moyen déterminé à l'aide d'un granulomètre Coulter LS 100 (Coultronics) est de 6, 57 μm ± 0,25.
Stabilité : A l'état de suspension aqueuse, les microparticules peuvent être conservées au moins 5 mois à 4°C, à 20°C et à 45°C. Les microcapsules sont intactes, leur couleur n'est pas modifiée, et le surnageant est incolore.
Détermination de l'activité anti-radicalaire sur microcapsules fraîches en suspension dans l'eau distillée (concentration d'environ 0,7 % en poids sec de microcapsules), voir tableau II.

### Exemple 16

Fabrication de microcapsules constituées d'oligomères procyanidoliques de pépins de raisin (OPC PR) et de dextran co-réticulés par le chlorure de téréphtaloyle (CT).

Le protocole décrit à l'exemple 15 est appliqué en utilisant comme phase aqueuse une solution renfermant 10 % d'OPC PR (Leucocianidine, INDENA) et 5 % de dextran (average mol. 41600, Sigma) dans le tampon pH 11.

On obtient un sédiment de couleur chamois formé de belles microcapsules indépendantes, transparentes, à paroi épaisse de diamètre 5-15 $\mu$m. A l'état de suspension aqueuse, ces microcapsules peuvent être conservées au moins 6 semaines à 20°C. Le surnageant reste incolore.

### Exemple 17

Fabrication de microcapsules à partir d'extrait sec de myrtille et de chlorure de téréphtaloyle (CT).

On utilise un extrait sec hydroalcoolique de myrtille contenant des anthocyanosides en quantité correspondant à 15 % d'anthocyanidines (INDENA).

Le protocole décrit à l'exemple 1 est appliqué en utilisant comme phase aqueuse une solution à 10 % de cet extrait dans le tampon pH 11.

On obtient un sédiment de couleur rouge lie de vin, formé de belles microcapsules de 2 à 15 $\mu$m de diamètre, transparentes et à membrane nettement visible. Les microcapsules se remettent très facilement en suspension dans l'eau, donnant une suspension de couleur uniforme. Appliquée sur la peau, elle ne laisse aucune tache. Après sédimentation, la suspension laisse un surnageant incolore. Il en est de même après 15 jours de conservation à 20°C ou à 45°C.

### Exemple 18

Fabrication de microcapsules à partir de jus de raisin rouge et de chlorure de téréphtaloyle (CT).

On ajoute à 2 ml de jus de raisin ("Raisin rouge, pur jus" BONNETERRE) 1 ml de tampon pH 11.

Le protocole décrit à l'exemple 1 est appliqué en utilisant ce mélange comme phase aqueuse et en utilisant une vitesse d'agitation de 5 000 rpm.

On obtient un sédiment de couleur blanc cassé, formé de microcapsules à paroi fine, de 1 à 4 $\mu$m de diamètre.

### Exemple 19

Fabrication de microcapsules à partir de lyophilisat de jus de raisin rouge et de chlorure de téréphtaloyle (CT).

On prépare un lyophilisat de jus de raisin ("Raisin rouge, pur jus" BONNETERRE). On prépare ensuite une solution à 15 % de ce lyophilisat dans le tampon pH 11.

Le protocole décrit à l'exemple 1 est appliqué en utilisant ce mélange comme phase aqueuse.

On obtient un sédiment beige formé de belles microcapsules transparentes, à membrane bien visible, de diamètre 10 à 30 $\mu$m.

### Exemple 20

Fabrication de microcapsules à partir de jus de cassis et de chlorure de téréphtaloyle (CT).

On ajoute à 2 ml de jus de cassis ("Nectar de cassis", EDEN) 1 ml de tampon pH 11.

Le protocole décrit à l'exemple 1 est appliqué en utilisant ce mélange comme phase aqueuse et en utilisant une vitesse d'agitation de 5 000 rpm.

On obtient un sédiment de couleur rose pale, formé de microcapsules à paroi fine, de 2 à 5 $\mu$m de diamètre.

Exemple 21

Fabrication de microcapsules à partir de vin rouge et de chlorure de téréphtaloyle (CT).

On ajoute à 2 ml de vin rouge (Bordeaux: Château Grave de Blanquet, 1989) 1 ml de tampon pH 11.

Le protocole décrit à l'exemple 1 est ensuite reproduit en utilisant ce mélange comme phase aqueuse et en utilisant une vitesse d'agitation de 5 000 rpm.

On obtient un culot rose pâle formé de très petites microcapsules de 1 à 3 $\mu$m de diamètre.

Exemple 22

Fabrication de microcapsules à partir d'extrait de peau de raisin en poudre et de chlorure de téréphtaloyle (CT).

On prépare une solution à 10 % d'un extrait de peau de raisin en poudre ("Biocon grape skin extract powder", QUEST International) dans le tampon pH 11.

Le protocole décrit à l'exemple 1 est appliqué en utilisant ce mélange comme phase aqueuse et une solution à 2,5 % de CT.

On obtient un culot de couleur prune formé de microcapsules de diamètre 5-30 $\mu$m. Ces microcapsules se dispersent facilement dans l'eau, donnant une suspension de couleur lie de vin homogène ne tachant pas la peau. La suspension laisse après sédimentation un surnageant incolore.

Après un mois à 4°C ou à 45°C, le surnageant de la suspension aqueuse de microcapsules est toujours incolore, tandis que le culot de microcapsules a toujours une couleur prune.

Exemple 23

Fabrication de microcapsules à partir d'extrait sec de myrtille, d'oligomères procyanidoliques et de chlorure de téréphtaloyle (CT).

On utilise un extrait sec hydroalcoolique de myrtille contenant des anthocyanosides en quantité correspondant à 15 % d'anthocyanidines (INDENA).

On prépare une solution renfermant 10 % de cet extrait, et 5 % d'OPC PR (Leucocianidine, INDENA) dans le tampon pH 11.

Le protocole décrit à l'exemple 1 est appliqué en utilisant cette solution comme phase aqueuse.

On obtient un culot de microcapsules rouge framboise, de diamètre 5 à 15 $\mu$m, se dispersant facilement dans l'eau pour donner une suspension de couleur homogène. Après sédimentation, le surnageant est incolore.

Exemple 24

Fabrication de microcapsules constituées d'oligomères procyanidoliques de pépin de raisin (OPC PR) réticulés par le chlorure de téréphtaloyle (CT), et contenant une huile.

On prépare une solution à 10 % d' OPC PR (SARPAP) dans le tampon pH 11. On émulsionne 2 ml d'huile d'olive dans 12 ml de cette solution par agitation de 2 min à 5 000 rpm.

Puis, on abaisse la vitesse d'agitation à 3 000 rpm et on ajoute 60 ml de cyclohexane renfermant 5 % de Span 85$^{®}$.

Après 3 min d'agitation on ajoute à l'émulsion 80 ml d'une solution à 5 % (p/v) de CT dans un mélange de chloroforme : cyclohexane 1:4 (v/v) et l'agitation est maintenue pendant 30 min.

Le milieu réactionnel est dilué par addition de 50 ml de cyclohexane, puis les microcapsules sont séparées par centrifugation et lavées par remise en suspension successivement dans le cyclohexane, dans l'eau additionnée de 2 % de Tween 20$^{®}$, et finalement dans l'eau distillée.

On obtient des microcapsules de couleur chamois, flottant à la surface de l'eau. L'examen microscopique montre des vésicules sphériques contenant des gouttelettes brillantes réfringentes.

Exemple 25

Fabrication de microcapsules à partir d'oligomères procyanidoliques, de catalase et de chlorure de téréphtaloyle (CT).

On prépare une solution renfermant 10 % d'OPC PR (Leucocianidine, INDENA) et 3 % de catalase (de foie de bovin, C-10, SIGMA) dans le tampon pH 11.

Le protocole décrit à l'exemple 1 est appliqué en utilisant cette solution comme phase aqueuse.

On obtient de belles microcapsules de couleur chamois, de diamètre 10-30 μm.

Lorsqu'on prélève avec une spatule une petite quantité de microcapsules fraîchement préparées et qu'on les met au contact d'eau oxygénée à 110 volumes, on observe immédiatement une mousse abondante, ce qui montre que la catalase contenue dans les microcapsules conserve une activité enzymatique.

Exemple 26

Fabrication de microcapsules constituées d'acide caféique réticulé par le chlorure de téréphtaloyle (CT).

Dans 6 ml d'un tampon pH 9,8, on dissout 600 mg d'acide caféique (Sigma). On émulsionne cette solution dans 30 ml de cyclohexane additionné de 5 % de Span 85®, par agitation à 5 000 rpm. Après 5 min, on ajoute à l'émulsion 40 ml d'une solution à 5 % (p/v) de CT dans un mélange de chloroforme : cyclohexane 1:4 (v/v) et l'agitation est maintenue pendant 30 min. On ajoute ensuite 40 ml de cyclohexane au milieu réactionnel pour arrêter la réaction.

Après lavages, on obtient un sédiment de couleur blanc crème, formé de microcapsules de diamètre 2-10 μm, à contenu clair, et à paroi nette. Après lyophilisation, on obtient une poudre blanche. L'examen microscopique de la poudre réhydratée montre des microcapsules intactes et sphériques.

Exemple 27

Fabrication de microcapsules constituées de phloroglucinol réticulé par le chlorure de téréphtaloyle

Le protocole décrit à l'exemple 26 est appliqué en utilisant comme phase aqueuse une solution renfermant 10 % de phloroglucinol (Sigma) dans le tampon pH 11. On obtient un sédiment blanc formé de microcapsules à contenu granuleux, de diamètre 2-10 μm.

Exemple 28

Fabrication de microcapsules constituées d'acide protocatéchique réticulé par le chlorure de téréphtaloyle

Le protocole décrit à l'exemple 26 est appliqué en utilisant comme phase aqueuse une solution renfermant 10 % d'acide protocatéchique (Sigma) dans le tampon pH 11. On obtient un sédiment blanc, formé de microcapsules à contenu clair de diamètre 2-10 μm.

Exemple 29

Fabrication de microcapsules constituées de DL-3,4-dihydroxyphénylalanine (DL-DOPA) réticulée par le chlorure de téréphtaloyle

Le protocole décrit à l'exemple 26 est appliqué en utilisant comme phase aqueuse une solution renfermant 10 % de DL-DOPA (Sigma) dans le tampon pH 9,8. On obtient un sédiment de couleur kaki, formé de microcapsules à contenu granuleux de diamètre 2-10 μm.

Exemple 30

Fabrication de microcapsules constituées de curcumine réticulée par le chlorure de téréphtaloyle

Le protocole décrit à l'exemple 26 est appliqué en utilisant comme phase aqueuse une solution renfermant 10 % de curcumine (Sigma) dans la soude 1M. On obtient un sédiment de couleur jaune vif, formé de microcapsules à contenu clair, de diamètre 2-10 μm.

Exemple 31

Fabrication de microcapsules constituées d'acide ellagique réticulé par le chlorure de téréphtaloyle

Le protocole décrit à l'exemple 26 est appliqué en utilisant comme phase aqueuse une solution renfermant 10% d'acide ellagique (Sigma) dans le tampon pH 11. On obtient un sédiment de couleur brun-vert, formé de microcapsules de diamètre 10-15 $\mu$m.

### Exemple 32

#### Fabrication de microcapsules à partir de silymarine

Le protocole décrit à l'exemple 26 est appliqué en utilisant comme phase aqueuse une solution renfermant 10 % de silymarine (Silimarina /S Indena) dans la soude 3M. On obtient un sédiment orange clair, formé de microcapsules de forme irrégulière, de diamètre 2-8 $\mu$m.

### Exemple 33

#### Fabrication de microcapsules à partir de rutine

Le protocole décrit à l'exemple 26 est appliqué en utilisant comme phase aqueuse une solution renfermant 10 % de rutine (trihydrate, Sigma) dans la soude 1M. On obtient un sédiment orange, formé de microcapsules sphériques, de diamètre 3-5 $\mu$m.

### Exemple 34

#### Fabrication de microcapsules à partir d'un extrait de Gingko biloba

Le protocole décrit à l'exemple 26 est appliqué en utilisant comme phase aqueuse une solution renfermant 10 % d'extrait sec de Gingko biloba (Indena) dans la soude 3M. Après dispersion dans l'eau distillée, on obtient un sédiment de faible volume, compact, de couleur beige, formé de microcapsules de forme irrégulière, de diamètre moyen 10,79 $\mu$m (détermination au granulomètre Coulter LS 100$^{®}$, Coultronics).

### Exemple 35

#### Fabrication de microcapsules à partir d'un extrait de Gingko biloba avec addition d'un polyéthylèneglycol

Le protocole décrit à l'exemple 26 est appliqué en utilisant comme phase aqueuse une solution renfermant 10 % d'extrait sec de Gingko biloba (Indena) dans de la soude 3M contenant 5 % v/v de polyéthylèneglycol [PEG 200, Sigma]. Après dispersion dans l'eau distillée, on obtient un volumineux sédiment de couleur beige, formé de microcapsules parfaitement sphériques, de diamètre moyen 27,94 $\mu$m (détermination au granulomètre Coulter LS 100$^{®}$, Coultronics).

Cet exemple montre que l'addition d'un polyéthylèneglycol à la phase aqueuse permet d'augmenter l'hydrophilie des microcapsules.

### Exemple 36

#### Fabrication de microcapsules à partir d'oligomères procyanidoliques de pépins de raisins (OPC-PR) réticulés par le chlorure de sébacoyle et renfermant une huile

On prépare une phase aqueuse contenant 15% d'OPC PR (INDENA) dans le tampon de pH 11.

On prépare une phase huileuse par addition de 0,3 ml de chlorure de sébacoyle à 6 ml d'huile d'olive.

On émulsionne 3 ml de la phase huileuse, utilisée comme phase dispersée, dans 10 ml de la phase aqueuse, par agitation à 5000 rpm.

On laisse la réaction se développer pendant 60 minutes.

Le milieu est dilué par addition de 100 ml d'eau distillée. Puis, les microcapsules sont séparées et lavées à l'eau.

On obtient un surnageant de couleur blanc crème formé de microcapsules de diamètre 10-30 $\mu$m. L'examen microscopique montre que chaque gouttelette d'huile est emprisonnée dans une membrane. Les microcapsules, mises en dispersion dans l'eau, sont stables au moins 2 mois à l'étuve à 45°C : les microcapsules sont intactes, et l'eau de dispersion est incolore.

Exemple 37

Mise en évidence de l'activité anti-radicalaire des microcapsules selon l'invention

Différentes microcapsules selon l'invention ont été testées pour leur capacité à piéger les radicaux libres.

Le test utilisé était le test dit "test NBT" (nitrobleu de tetrazolium), dont le principe utilise la réaction de réduction du NBT en une substance colorante bleue, le bleu de formazan, par des anions superoxydes $O_2^-$ formés à partir du système enzymatique hypoxanthine - xanthine oxydase. La xanthine oxydase catalyse l'oxydation de l'hypoxanthine en xanthine puis en acide urique avec formation d'anions superoxydes. Si le composé testé, introduit dans le milieu réactionnel, possède une activité anti-radicalaire, il "piégera" les anions superoxydes et, de ce fait, réduira la formation de colorant bleu.

Ce test est bien connu de l'homme de l'art et a été utilisé et décrit notamment par : DE LAMIRANDE E. et al., Fertility and sterility, 1993, 59 (6), 1291-5 ; RAMESH Chander et al., Biochemical Pharmacology, 1992, 44 (1), 180-183.

La formation du bleu de formazan est déterminée colorimétriquement, par exemple au moyen d'un spectrophotomètre UV - visible, à la longueur d'onde de 560 nm.

La formation de ce colorant en fonction du temps est linéaire pendant les cinq premières minutes. L'activité réductrice de l'anion superoxyde sera donc exprimée par la pente de la droite obtenue. Cette pente, rapportée à celle d'un témoin ne comportant pas de "piégeur" de radicaux libres, permettra d'établir l'efficacité de l'effet piégeur du produit testé.

Mode opératoire

1 - Réactifs :

    [T] : Tampon TRIS-HCl 0,05 M pH 7,4 (TRIZMA PRE-SET pH crystals, SIGMA)
    [N] : Nitrobleu de Tétrazolium $10^{-3}$ M (Grade III, SIGMA) préparé dans [T]
    [H] : Hypoxanthine $0,5.10^{-2}$ M, préparée dans [T]
    [X.O] : Xanthine Oxydase 1,67 U/ml préparée dans [T]

Les solutions d'hypoxanthine et de xanthine oxydase sont préparées extemporanément ; la solution de N.B.T. peut être conservée plusieurs jours au réfrigérateur à +4°C et à l'obscurité.

2 - Préparation des echantillons :

Les produits essayés, c'est-à-dire les microcapsules de polyphénols végétaux réticulés selon l'invention, ont été dispersés dans la solution [T] à la concentration de 1 mg de produit par ml de tampon. On a ainsi utilisé soit des microcapsules préalablement lyophilisées que l'on a dispersées directement dans la solution tampon, soit des microcapsules séparées par centrifugation d'une suspension fraîchement obtenue selon les procédés décrits dans les exemples précédents.

A titre d'essai comparatif, on a également préparé une solution des mêmes polyphénols végétaux non réticulés, à une concentration de 1 mg/ml dans [T].

3 - Matériel :

Les analyses ont été effectuées sur un spectrophomètre de type UV - visible relié à un enregistreur. La longueur d'onde est réglée à 560 nm.

4 - Mise en oeuvre :

A chaque analyse de produit, trois séries de cuves pour spectrophotomètre sont préparées à partir des réactifs et des dispersions ou solutions de produits à essayer décrits précédemment. Le contenu de ces cuves est résumé dans le tableau I ci-dessous, dans lequel les quantités de produits ou réactifs sont exprimées en ml.

TABLEAU I

| | ESSAI | REACTIFS (ml) | | | |
|---|---|---|---|---|---|
| | | [T] | [N] | [H] | [E] |
| CUVES 0 | 0 % | 2,4 | 0,1 | 0,5 | --- |
| CUVES 1 | 100 % | 2,3 | 0,1 | 0,5 | --- |
| CUVES 2 | X | 2,2 | 0,1 | 0,5 | 0,1 |

[E] : échantillon à étudier.

Après homogénéisation, ces solutions sont équilibrées avec l'air ambiant, à 25° + ou - 1°C pendant 20 min. Au bout de 20 min, chacune de ces cuves fait l'objet d'un enregistrement spectrophotométrique pendant 5 min. La cuve 0 est enregistrée directement.

L'enregistrement des cuves 1 et 2 ne commence que sitôt après avoir disposé, dans le milieu réactionnel, 0,1 ml de la solution de xanthine oxydase.

Plus précisément, l'enregistrement des cuves 2 sera effectué à un temps précis, par exemple à 2, 3, 4 ou 5 min après l'introduction de l'enzyme. On aura pris soin, avant d'effectuer la lecture photométrique, d'éliminer, par exemple par centrifugation, les microcapsules de la solution contenue dans les cuves. Il est donc nécessaire, en début d'expérience de préparer un nombre suffisant de cuves 2 pour réaliser les lectures photométriques aux différents temps désirés.

L'enregistrement spectrophotométrique des cuves 0 donne la pente moyenne P0 qui est très voisine de 0. Celui des cuves 1 donne la pente moyenne P1 et représente le 100 %, c'est-à-dire l'effet maximal de l'anion superoxyde $0_2^-$. Enfin, celui des cuves 2 donne la pente moyenne P2 qui est intermédiaire entre P0 et P1. Cette pente traduit l'inhibition due à "l'effet piège" des produits analysés.

Le pourcentage d'inhibition "A" est donné par la relation :

$$A = \frac{(P1-P0)-P2}{P1-P0} \times 100$$

Pour chaque analyse, trois essais ont été effectués ; ces essais ont donné lieu à des moyennes. L'ensemble de ces moyennes sont rassemblées dans le tableau II ci-dessous.

TABLEAU II

| Produit testé N° exemple | OPC PR non réticulé | Ex. 1 | Ex. 3 | Ex. 5 | Ex. 6 | Ex. 9 | Ex. 11 | Ex. 15 |
|---|---|---|---|---|---|---|---|---|
| Activité anti-radicalaire A % | 91 ± 2 | 81±4 | 51±1 | 71±2 | 83±1 | 79±3 | 64±5 | 78±2 |

OPC PR = Oligomére ProCyanidolique de Pépins de Raisin.

Remarque :

Toutes les microcapsules testées avaient été préalablement lyophilisées, à l'exception des microcapsules de l'exemple 15 qui avaient été séparées par centrifugation d'une suspension fraîchement obtenue.

Ces résultats démontrent que les polyphénols végétaux réticulés selon l'invention, sous forme de microcapsules, ont, de manière surprenante, conservé une activité anti-radicalaire sensiblement de même importance.

Ainsi, les microcapsules selon l'invention ont conservé l'activité des polyphénols végétaux dont elles sont issues et peuvent être utilisées avantageusement dans ce but dans différentes compositions, notamment cosmétiques ou pharmaceutiques, tout en présentant une très grande stabilité.

Diverses formulations de compositions cosmétiques ou pharmaceutiques, notamment dermatologiques, sont données ci-après.

Exemple 38

Composition de base pour composition cosmétique ou pharmaceutique.

On disperse des microcapsules lyophilisées d'OPC réticulés telles qu'obtenues selon l'un quelconque des exemples précédents 1 à 8, 15 ou 16, à la concentration de 0.1 % dans un excipient gras, par exemple un excipient gras disponible dans le commerce de dénomination commerciale Cold Cream naturel des Laboratoires Roche Posay. On constate que les microcapsules se répartissent très facilement dans l'excipient et donnent une préparation d'aspect homogène présentant une très légère teinte rosée.

Cette composition est particulièrement stable.

Exemple 39

Composition sous forme d'un gel protecteur.

Cette composition présente les ingrédients suivants en pourcentage en poids :

| | % en poids |
| --- | --- |
| Eau | 64,30 |
| Conservateur imidazolin urée | 0,20 |
| Carbomer 940$^®$ | 0,50 |
| Hydroxyde de sodium à 10 % | 1,90 |
| Alcool 96° | 20,00 |
| Suspension de microcapsules d'oligomères procyanidoliques de pépins de raisin à 0,7 %, selon l'exemple 15 | <u>14,30</u> |
| | 100,00 |

Mode opératoire de préparation :

On prépare d'abord le gel de Carbomer 940 de façon classique, à avoir que l'on dissout le conservateur dans l'eau, puis on disperse le carbomer 940. Ensuite, on neutralise par la solution de soude sous agitation. On ajoute l'alcool, et enfin, on ajoute sous agitation la suspension de microcapsules en obtenant ainsi une composition sous forme d'un gel.

Ce gel s'applique sur la peau, par exemple du visage ou des mains, et permet ensuite l'application d'une crème et/ou d'un maquillage.

Exemple 40

Composition cosmétique sous forme de crème de jour

Cette composition se compose de trois phases A, B, C, respectivement suivantes :

| Phase A : | % en poids |
|---|---|
| Steareth 2 | 0,8 |
| Steareth 21 | 2,2 |
| Cétyl palmitate | 1,5 |
| Alcool stéarylique | 1,8 |
| Glycéryl monostéarate | 1,5 |
| Caprylic capric triglycéride | 4,6 |
| Acide stéarique | 2,6 |
| Perhydrosqualène | 11,0 |
| Parabens | 0,5 |
| Phase B | |
| Eau | 57,20 |
| Méthyl paraben | 0,15 |
| Carbomer 940 | 0,20 |
| Phase C | |
| Eau | 1,26 |
| Sodium hydroxyde | 0,14 |
| Phase D | |
| Suspension de microcapsules d'oligomères procyanidoliques de pépins de raisin (OPC PR) à 0,7 % selon l'exemple 15 | 14,3 |
| Phase E | |
| Parfum | <u>0,25</u> |
| | 100,00 |

Mode opératoire de préparation :

On prépare la phase grasse (phase A) de manière classique en mélangeant les ingrédients indiqués ci-dessus, puis on chauffe à 85°C.

On prépare ensuite la phase aqueuse (phase B) de la manière suivante :

- on chauffe l'eau à 85°C, dans laquelle on dissout le conservateur,
- ensuite, on disperse le carbomer 940, et on maintient la température à 85°C.

Dans un mélangeur agitateur de type YSTRAL®, on verse lentement la phase aqueuse B sur la phase grasse A. On poursuit l'agitation tout en laissant refroidir le mélange. Lorsque la température atteint 70°C, on neutralise avec la phase C (solution de soude). Puis, à 40°C, on ajoute la suspension de microcapsules d'OPC PR (phase D). On poursuit toujours l'agitation tout en laissant refroidir, et on parfume à 35°C (phase E). On agite encore, jusqu'à ce que l'émulsion huile dans eau obtenue atteigne la température ambiante.

La stabilité de cette crème a été déterminée. Conservée à l'étuve à 40°C, elle n'a subi aucune modification de couleur après un mois, contrairement à un échantillon témoin de crème préparée à partir des phases A, B, C, E et contenant des OPC non réticulés, placé dans les mêmes conditions.

On obtient ainsi une crème de jour qui peut être appliquée régulièrement sur la peau, par exemple du visage ou des mains.

**Revendications**

1. Microcapsules, caractérisées en ce qu'elles comprennent une paroi formée d'un ou plusieurs polyphénols végétaux réticulés, en particulier au moyen d'une réticulation interfaciale entre le ou les polyphénols végétaux et un agent réticulant, de préférence un halogénure de diacide, en particulier un chlorure de diacide.

2. Microcapsules selon la revendication 1, caractérisées en ce qu'elles comprennent une protéine ou un polysaccharide, ou un polyalkylèneglycol ou un mélange quelconque de ces substances.

3. Microcapsules selon la revendication 2, caractérisées en ce que leur paroi comprend une protéine et/ou un polysaccharide et/ou un polyalkylèneglycol co-réticulé avec le ou les polyphénols végétaux précités.

4. Microcapsules selon l'une des revendications 2 ou 3, caractérisées en ce que la protéine précitée est douée d'une activité biologique spécifique, telle qu'une activité enzymatique, comme par exemple la catalase, la superoxyde dismutase, ou la glutathion peroxydase, cette activité pouvant s'ajouter à l'activité propre du ou des polyphénols végétaux.

5. Microcapsules selon l'une des revendications 1 à 4, caractérisée en ce que les polyphénols végétaux précités sont des polyphénols végétaux monocycliques ou polycycliques, tels que les flavonoïdes, les isoflavonoïdes, les néoflavonoïdes, les tanins galliques et les tanins ellagiques, le catéchol et ses dérivés tels que la DL-3,4-dihydroxyphénylalanine ou DL-DOPA, ou les catécholamines telles que la 3-hydroxytyramine ou dopamine, ou le phloroglucinol, ou les acides phénols tels que l'acide caféique, l'acide dihydrocaféique, l'acide protocatéchique, l'acide chlorogénique, l'acide isochlorogénique, l'acide gentisique, l'acide homogentisique, l'acide gallique, l'acide hexahydroxydiphénique, l'acide ellagique, l'acide rosmarinique, l'acide lithospermique, ou les dérivés des acides phénols, en particulier leurs esters ou leurs hétérosides, ou la curcumine, ou les coumarines polyhydroxylées, ou les lignanes ou les néolignanes polyhydroxylés, ou un mélange contenant un ou plusieurs polyphénols végétaux ou leurs dérivés, comme la silymarine, tous les polyphénols précités pouvant être utilisés sous forme de préparations obtenues à partir de plantes ou de parties de plantes, telles que des extraits, des teintures, des jus de fruits, des vins.

6. Microcapsules selon l'une des revendications précédentes, caractérisées en ce que les flavonoïdes précités peuvent être choisis parmi le groupe consistant d'une flavone, telle que l'apigénol, le lutéolol, un flavonol comme la quercétine, le kaempferol, ou un hétéroside de flavone ou de flavonol, comme la rutine et ses dérivés, une flavanone comme la flavanone, la naringénine, l'hespérétine, ou un hétéroside de flavanone comme la naringine, l'hespéridine, la diosmine, ou un dérivé de flavanone comme le diosmoside, ou un biflavonoïde, ou un dimère de flavone ou de flavonone comme l'amentoflavone, ou une chalcone telle que l'isoliquirtigénine ou l'hespéridine méthylchalcone, un flavanonol tel que le taxifoliol ou une substance dérivée de taxifoliol telle que la silybine, la silychristine, la silydianine, un flavan-3-ol tel que la (+) catéchine, la (-) épicatéchine, un polymère formé d'unités de structure de base flavan-3-ol, généralement désigné sous le nom de "proanthocyanidine" ou sous l'expression "tanin condensé", en particulier un oligomère comprenant de 2 à 8 de ces unités, appelé généralement "oligomère procyanidolique" (OPC), un anthocyanoside comme le malvoside ou un mélange contenant un ou plusieurs flavonoïdes, en particulier sous la forme d'extraits de fruits ou d'extraits de plantes ou de parties de plantes.

7. Microcapsules selon la revendication 6, caractérisées en ce que les mélanges contenant des flavonoïdes peuvent comprendre des mélanges de citroflavonoïdes extraits de divers Citrus (Rutacées), un mélange de flavonoïdes extrait de Silybum marianum (Composées) ou la silymarine, les extraits de Gingko biloba (Ginkgoacées), les extraits riches en anthocyanosides de myrtille, de fruits de cassis, de peaux de raisin, de feuille de vigne rouge, les jus de fruits tels que les jus de raisin, de cassis, tels quels ou concentrés ou desséchés notamment par nébulisation ou lyophilisation, les vins rouges, tels quels ou concentrés ou desséchés, ou leurs divers mélanges.

8. Microcapsules selon l'une des revendications 2 à 7, caractérisées en ce que la protéine précitée peut être choisie parmi le groupe consistant des albumines comme la sérumalbumine, l'ovalbumine, l'alpha-lactalbumine, les globulines, le fibrinogène, la caséine, les protéines végétales telles que les protéines du soja, les glutélines qui de préférence auront été dégradées, les scléroprotéines solubilisées, le collagène, l'atélocollagène, la gélatine, les hydrolysats de gélatine, les peptones, l'hémoglobine, les enzymes telles que la catalase, la superoxyde dismutase, la glutathion proxydase, des mélanges contenant des protéines hydrophiles, tels que le lait entier ou écrémé totalement ou partiellement, le lait en poudre, le lait condensé, les protéines du lactosérum, la farine de soja, les mélanges d'atélocollagène et de glycosaminoglycanes.

9. Microcapsules selon l'une des revendications 2 à 8, caractérisées en ce que le polysaccharide précité but être choisi parmi le groupe consistant des dextrans, de l'acide alginique et ses sels hydrosolubles, en particulier l'alginate de sodium, les gommes végétales, les carraghénanes, les pectines, les dérivés solubles d'amidon, les dérivés solubles de cellulose, les glycosaminoglycanes.

10. Microcapsules selon l'une des revendications 2 à 9, caractérisées en ce que le polyalkylèneglycol précité peut être choisi parmi le groupe consistant des polyéthylèneglycols et des polypropylèneglycols.

11. Microcapsules selon l'une des revendications précédentes, caractérisées en ce qu'elles sont préparées par réticulation interfaciale à partir d'une émulsion dont la phase aqueuse contient de 1 % à 40 %, de préférence entre 1 et 20 % en poids de polyphénols végétaux par rapport au poids total de la phase aqueuse ; lorsque une protéine et/ou un polysaccharide et/ou un polyalkylèneglycol précité est présent, la concentration totale dans la phase aqueuse de cette ou de ces substance(s) est comprise avantageusement entre 0,1 et 30 % en poids, de préférence entre 1 et 10 % en poids, par rapport au poids total de la phase aqueuse.

12. Microcapsules selon l'une des revendications précédentes, caractérisées en ce qu'elles contiennent une ou plusieurs substances actives hydrosolubles, liposolubles ou insolubles, à l'état de solution, de suspension ou d'émulsion, en particulier une substance minérale réflectrice des radiations solaires, de préférence insoluble, une huile végétale ou une solution huileuse contenant une substance active liposoluble tel qu'un filtre solaire liposoluble.

13. Microcapsules selon la revendication 12, caractérisées en ce que la substance active précitée incorporée dans les microcapsules est choisie parmi le groupe consistant en une substance minérale réflectrice des radiations solaires, telle qu'un oxyde de fer, l'oxyde de titane, l'oxyde de zinc, le talc, le kaolin, une huile végétale telle qu'une huile de germes de céréales ou une huile de foie de poisson désodorisée, ou une solution huileuse d'une substance liposoluble telle que la vitamine A, la vitamine D2, la vitamine E ou tocophérol, un acide gras essentiel tel que l'acide linoléique, l'acide linolénique, l'acide arachidonique, une céramide, un dérivé liposoluble d'acide ascorbique tel que le palmitate d'ascorbyle, ou un filtre solaire liposoluble tel qu'un ester cinnamique, un ester paraaminobenzoïque, un ester salicylique, une benzophénone, le benzylidène camphre et ses dérivés, un dérivé du dibenzoylméthane, un benzimidazole, ou une substance photoactive telle que le bergaptène ou tout autre dérivé du psoralène, ou encore un mélange contenant plusieurs substances actives.

14. Microcapsules selon l'une des revendications précédentes, caractérisées en ce qu'elles présentent un diamètre compris entre 0,1 μm (micromètre) et 3 mm.

15. Procédé de fabrication de microcapsules, caractérisé en ce qu'on réalise une réticulation interfaciale d'une émulsion de type eau-dans-l'huile, comprenant les étapes essentielles suivantes :

> a) on prépare une phase aqueuse contenant le polyphénol végétal ou le mélange de polyphénols végétaux à réticuler,
>
> b) on prépare une phase hydrophobe, contenant éventuellement un ou plusieurs agents tensio-actifs,
>
> c) on émulsionne ladite phase aqueuse dans la phase hydrophobe précitée, de sorte que la phase hydrophobe constitue la phase continue dans laquelle la phase aqueuse forme la phase dispersée,
>
> d) on ajoute à l'émulsion ainsi obtenue un agent réticulant dissous dans un liquide miscible à la phase hydrophobe, sous agitation, pour réaliser une réticulation interfaciale de l'agent réticulant et du ou des polyphénols végétaux contenus dans la phase aqueuse,
>
> e) on maintient l'agitation pendant un temps de réaction convenable pour obtenir une réticulation suffisante conduisant à la formation de microcapsules dont la paroi comprend le ou les polyphénols végétaux réticulés par l'agent réticulant,
>
> f) on recueille les microcapsules ainsi formées, par tout moyen approprié.

16. Procédé de fabrication de microcapsules, caractérisé en ce qu'on réalise une réticulation interfaciale d'une émulsion de type huile-dans-eau comprenant les étapes essentielles suivantes :

a) on prépare une phase hydrophobe dans laquelle on dissout l'agent réticulant,

b) on prépare une phase aqueuse contenant le polyphénol végétal ou le mélange de polyphénols végétaux à réticuler, et éventuellement un ou plusieurs agents tensio-actifs,

c) on émulsionne la phase hydrophobe dans la phase aqueuse précitée de sorte que la phase aqueuse constitue la phase continue dans laquelle la phase hydrophobe forme la phase dispersée,

d) on maintient l'ensemble sous agitation pendant un temps de réaction convenable pour obtenir une réticulation suffisante conduisant à la formation de microcapsules dont la paroi comprend le ou les polyphénols végétaux réticulés par l'agent réticulant,

e) on recueilie les microcapsules ainsi formées, par tout moyen approprié.

**17.** Procédé selon la revendication 15 ou 16, caractérisé en ce qu'on ajoute à la phase aqueuse, lors de sa préparation, une protéine ou un polysaccharide, ou un polyalkylèneglycol ou un mélange quelconque de ces substances.

**18.** Procédé selon l'une des revendications 15 à 17, caractérisé en ce qu'on réalise des lavages des microcapsules formées, afin d'éliminer l'agent réticulant en excès ainsi que le ou les polyphénols végétaux n'ayant pas réagi ou non encapsulés dans les microcapsules.

**19.** Procédé selon l'une des revendications 15 à 18, caractérisé en ce que les microcapsules sont lyophilisées.

**20.** Procédé selon l'une des revendications 15 à 19, caractérisé en ce que l'agent réticulant comprend un halogènure de diacide, en particulier un chlorure de diacide, de préférence choisi parmi le groupe consistant d'un chlorure de diacide aliphatique ou aromatique, tel que le chlorure de sébacoyle, le chlorure de succinyle, le chlorure d'adipoyle, le chlorure de téréphtaloyle, le chlorure de glutaryle.

**21.** Procédé selon l'une des revendications 15 à 20, caractérisé en ce que la concentration en halogénure de diacide est comprise entre 0,2 % et 10 % en poids du poids total du milieu réactionnel.

**22.** Procédé selon l'une des revendications 15 à 21, caractérisé en ce que le pH de la réaction est compris entre 8 et 14, et encore mieux entre 9 et 12 et peut être assuré par une solution tampon ou une solution d'un agent alcalin tel que la soude ou la potasse.

**23.** Procédé selon l'une des revendications 15 à 22, caractérisé en ce que la phase hydrophobe non miscible à la phase aqueuse précitée est réalisée à partir de substances liquides hydrophobes choisies parmi le groupe consistant des hydrocarbures halogénés ou non, tels que le cyclohexane, le chloroforme ou le dichlorométhane, des esters d'acides gras, tels que le myristate d'isopropyle ou l'oléate d'éthyle, des mélanges d'esters d'acides gras tels que par exemple le produit Dragoxat$^®$, des huiles végétales, telles que l'huile d'olive, l'huile d'amande douce ou l'huile d'arachide, des huiles minérales, telles qu'une huile de paraffine, et tout mélange de ces substances liquides hydrophobes.

**24.** Procédé selon l'une des revendications 15 à 23, caractérisé en ce qu'on incorpore à la phase devant être dispersée, constituant la phase liquide à encapsuler, une ou plusieurs substances actives, à l'état de solution, de suspension ou d'émulsion, en particulier une substance minérale réflectrice des radiations solaires, de préférence insoluble, une huile vu une solution huileuse de substance liposoluble, telle qu'un filtre solaire liposoluble.

**25.** Composition, en particulier composition cosmétique ou pharmaceutique, notamment dermatologique, composition diététique, ou composition alimentaire, caractérisée en ce qu'elle comprend des microcapsules à paroi de polyphénols végétaux réticulés, telles que définies à l'une quelconque des revendications 1 à 14, ou obtenues par la mise en oeuvre du procédé selon l'une quelconque des revendications 15 à 24.

**26.** Composition selon la revendication 25, caractérisée en ce que la concentration en microcapsules à paroi de polyphénols végétaux réticulés est comprise entre 0,01 et 10 % en poids du poids total de la composition finale, encore mieux entre 0,1 et 5 % en poids de la composition finale.

**27.** Composition selon la revendication 25 ou 26, caractérisée en ce que les microcapsules à paroi de polyphénols végétaux réticulés se présentent sous forme de microcapsules brutes de fabrication dites fraîches.

28. Composition selon la revendication 25 ou 26, caractérisée en ce que les microcapsules à paroi de polyphénols végétaux réticulés se présentent sous forme desséchée, notamment par lyophilisation.

29. Composition selon l'une des revendications 25 à 28, caractérisée en ce qu'elle est destinée à prévenir le vieillissement cutané, notamment le vieillissement actinique.

30. Méthode de traitement cosmétique d'un être humain pour la prévention du vieillissement cutané, notamment du vieillissement actinique dû généralement aux radicaux libres, caractérisée en ce qu'on applique une quantité efficace de microcapsules à paroi de polyphénols végétaux réticulés, telles que définies à l'une quelconque des revendications 1 à 14, éventuellement incluses dans un excipient, véhicule ou support cosmétiquement ou pharmaceutiquement acceptable, sur les zones de la peau ou des cheveux sensibles à l'action des radicaux libres, notamment les radicaux libres résultant d'une exposition actinique.

31. Méthode de traitement cosmétique selon la revendication 30, caractérisée en ce que la concentration en microcapsules est habituellement comprise entre 0,01 et 10 % en poids, et de préférence entre 0,1 et 5 % en poids de la composition contenant les microcapsules.

32. Procédé de préparation d'une composition incorporant un ou plusieurs polyphénols végétaux, caractérisé en ce qu'en vue de prévenir toute altération, notamment toute modification de coloration de la composition au cours du temps, tout en conservant l'activité des polyphénols végétaux, notamment l'activité biologique, ces derniers sont incorporés dans ladite composition sous forme de microcapsules telles que définies selon l'une quelconque des revendications 1 à 14 ou obtenues par la mise en oeuvre du procédé selon l'une quelconque des revendications 15 à 24.

33. Procédé selon la revendication 32, caractérisé en ce que la composition est choisie parmi le groupe consistant d'une composition cosmétique ou pharmaceutique, notamment dermatologique, d'une composition diététique, ou d'une composition alimentaire.

34. Procédé selon la revendication 32 ou 33, caractérisé en ce que l'activité conservée par les polyphénols végétaux, en particulier par les flavonoïdes, est une activité anti-radicalaire et/ou anti-oxydante.

## Claims

1. Microcapsules, characterized in that they comprise a wall formed of one or more plant polyphenols crosslinked in particular by means of interfacial crosslinking between the plant polyphenol or polyphenols and a crosslinking agent, preferably a diacid halide and particularly a diacid chloride.

2. Microcapsules according to Claim 1, characterized in that they comprise a protein, a polysaccharide, a polyalkylene glycol or any mixture of these substances.

3. Microcapsules according to Claim 2, characterized in that their wall comprises a protein and/or a polysaccharide and/or a polyalkylene glycol co-crosslinked with the abovementioned plant polyphenol or polyphenols.

4. Microcapsules according to one of Claims 2 or 3, characterized in that the abovementioned protein possesses a specific biological activity such as an enzymatic activity, examples being catalase, superoxide dismutase or glutathione peroxidase, it being possible for this activity to add to the inherent activity of the plant polyphenol or polyphenols.

5. Microcapsules according to one of Claims 1 to 4, characterized in that the abovementioned plant polyphenols are monocyclic or polycyclic plant polyphenols such as flavonoids, isoflavonoids, neoflavonoids, gallotannins and ellagitannins, catechol and derivatives thereof such as DL-3,4-dihydroxyphenylalanine or DL-DOPA, catecholamines such as 3-hydroxytyramine or dopamine, phloroglucinol phenolic acids such as caffeic acid, dihydrocaffeic acid, protocatechuic acid, chlorogenic acid, isochlorogenic acid, gentisic acid, homogentisic acid, gallic acid, hexahydroxydiphenic acid, ellagic acid, rosmarinic acid or lithospermic acid, phenolic acid derivatives, particularly their esters or their heterosides, curcumin, polyhydroxylated coumarins, polyhydroxylated lignans or neolignans, or a mixture containing one or more plant polyphenols or derivatives thereof, such as silymarin, it being possible for all the abovementioned polyphenols to be used in the form of preparations obtained from plants or parts of plants, such as extracts, tinctures, fruit juices or wines.

6. Microcapsules according to one of the preceding claims, characterized in that the abovementioned flavonoids can be selected from the group consisting of a flavone such as apigenol or luteolol, a flavonol such as quercetin or kaempferol, a flavone or flavonol heteroside such as rutin and derivatives thereof, a flavanone such as flavanone, naringenin or hesperetin, a flavanone heteroside such as naringin, hesperidin or diosmin, a flavanone derivative such as diosmoside, a biflavonoid, a flavone or flavonone dimer such as amentoflavone, a chalcone such as isoliquirtigenin or hesperidin methylchalcone, a flavanonol such as taxifoliol or a substance derived from taxifoliol, like silybin, silychristin or silydianin, a flavan-3-ol such as (+)-catechin or (-)-epicatechin, a polymer formed of flavan-3-ol basic structural units, which is generally known by the name of "proanthocyanidin" or by the expression "condensed tannin", in particular an oligomer comprising from 2 to 8 of these units, which is generally called a "procyanidolic oligomer" (PCO), or an anthocyanoside such as malvoside, or a mixture containing one or more flavonoids, particularly in the form of extracts of fruits or extracts of plants or of parts of plants.

7. Microcapsules according to Claim 6, characterized in that the mixtures containing flavonoids can comprise mixtures of citroflavonoids extracted from various Citrus (Rutaceae), a mixture of flavonoids extracted from Silybum marianum (Compositae), or silymarin, extracts of Ginkgo biloba (Ginkgoaceae), anthocyanoside-rich extracts of blueberry, blackcurrant fruits, grape skins or red vine leaf, fruit juices such as grape or blackcurrant juices, as such, concentrated or dehydrated, especially by spray-drying or lyophilization, red wines, as such, concentrated or dehydrated, or various mixtures thereof.

8. Microcapsules according to one of Claims 2 to 7, characterized in that the abovementioned protein can be selected from the group consisting of albumins such as serum albumin, ovalbumin or alpha-lactalbumin, globulins, fibrinogen, casein, vegetable proteins such as soya proteins, glutelins which will preferably have been degraded, solubilized scleroproteins, collagen, atelocollagen, gelatin, gelatin hydrolysates, peptones, haemoglobin, enzymes such as catalase, superoxide dismutase or glutathione peroxidase, mixtures containing hydrophilic proteins, such as whole milk or totally or partially skimmed milk, powdered milk or condensed milk, whey proteins, soya flour and mixtures of atelocollagen and glycosaminoglycans.

9. Microcapsules according to one of Claims 2 to 8, characterized in that the abovementioned polysaccharide can be selected from the group consisting of dextrans, alginic acid and water-soluble salts thereof, particularly sodium alginate, vegetable gums, carrageenans, pectins, soluble starch derivatives, soluble cellulose derivatives and glycosaminoglycans.

10. Microcapsules according to one of Claims 2 to 9, characterized in that the abovementioned polyalkylene glycol can be selected from the group consisting of polyethylene glycols and polypropylene glycols.

11. Microcapsules according to one of the preceding claims, characterized in that they are prepared by the interfacial crosslinking of an emulsion whose aqueous phase contains from 1% to 40% and preferably between 1 and 20% by weight of plant polyphenols, based on the total weight of the aqueous phase; if one of the abovementioned proteins and/or polysaccharides and/or polyalkylene glycols is present, the total concentration of this or these substance(s) in the aqueous phase is advantageously between 0.1 and 30% by weight and preferably between 1 and 10% by weight, based on the total weight of the aqueous phase.

12. Microcapsules according to one of the preceding claims, characterized in that they contain one or more water-soluble, liposoluble or insoluble active substances in the form of a solution, suspension or emulsion, in particular a preferably insoluble mineral substance which reflects solar radiation, a vegetable oil or an oily solution containing a liposoluble active substance such as a liposoluble sunscreen.

13. Microcapsules according to Claim 12, characterized in that the abovementioned active substance incorporated in the microcapsules is selected from the group consisting of a mineral substance which reflects solar radiation, such as an iron oxide, titanium oxide, zinc oxide, talc or kaolin, a vegetable oil such as a cereal germ oil, a deodorized fish liver oil, or an oily solution of a liposoluble substance such as vitamin A, vitamin D2, vitamin E or tocopherol, an essentially fatty acid such as linoleic acid, linolenic acid or arachidonic acid, a ceramide, a liposoluble ascorbic acid derivative such as ascorbyl palmitate, or a liposoluble sunscreen such as a cinnamic ester, a para-aminobenzoic ester, a salicylic ester, a benzophenone, benzylidenecamphor and derivatives thereof, a dibenzoylmethane derivative, a benzimidazole or a photoactive substance such as bergapten or any other psoralen derivative, or else a mixture containing several active substances.

14. Microcapsules according to one of the preceding claims, characterized in that they have a diameter of between 0.1 μm (micrometre) and 3 mm.

15. Process for the manufacture of microcapsules, characterized in that an emulsion of the water-in-oil type is subjected to interfacial crosslinking comprising the following essential steps:

a) an aqueous phase is prepared which contains the plant polyphenol or the mixture of plant polyphenols to be crosslinked,
b) a hydrophobic phase is prepared which contains one or more surfactants, if appropriate,
c) the said aqueous phase is emulsified in the abovementioned hydrophobic phase so that the hydrophobic phase forms the continuous phase in which the aqueous phase forms the disperse phase,
d) a crosslinking agent, dissolved in a liquid miscible with the hydrophobic phase, is added to the resulting emulsion, with agitation, in order to effect interfacial crosslinking of the crosslinking agent and the plant polyphenol or polyphenols contained in the aqueous phase,
e) agitation is maintained for a suitable reaction time to allow sufficient crosslinking, resulting in the formation of microcapsules whose wall comprises the plant polyphenol or polyphenols crosslinked by the crosslinking agent, and
f) the microcapsules thus formed are collected by any appropriate means.

16. Process for the manufacture of microcapsules, characterized in that an emulsion of the oil-in-water type is subjected to interfacial crosslinking comprising the following essential steps:

a) a hydrophobic phase is prepared in which the crosslinking agent is dissolved,
b) an aqueous phase is prepared which contains the plant polyphenol or the mixture of plant polyphenols to be crosslinked and, if appropriate, one or more surfactants,
c) the hydrophobic phase is emulsified in the abovementioned aqueous phase so that the aqueous phase forms the continuous phase in which the hydrophobic phase forms the disperse phase,
d) the whole is agitated for a suitable reaction time to allow sufficient crosslinking, resulting in the formation of microcapsules whose wall comprises the plant polyphenol or polyphenols crosslinked by the crosslinking agent, and
e) the microcapsules thus formed are collected by any appropriate means.

17. Process according to Claim 15 or 16, characterized in that a protein, a polysaccharide, a polyalkylene glycol or any mixture of these substances is added to the aqueous phase during its preparation.

18. Process according to one of Claims 15 to 17, characterized in that the microcapsules formed are washed in order to remove the excess crosslinking agent and the plant polyphenol or polyphenols which have not reacted or are not encapsulated in the microcapsules.

19. Process according to one of Claims 15 to 18, characterized in that the microcapsules are lyophilized.

20. Process according to one of Claims 15 to 19, characterized in that the crosslinking agent comprises a diacid halide, particularly a diacid chloride, which is preferably selected from the group consisting of an aliphatic or aromatic diacid chloride such as sebacoyl chloride, succinyl chloride, adipoyl chloride, terephthaloyl chloride or glutaryl chloride.

21. Process according to one of Claims 15 to 20, characterized in that the concentration of diacid halide is between 0.2% and 10% by weight of the total weight of the reaction medium.

22. Process according to one of Claims 15 to 21, characterized in that the reaction pH is between 8 and 14 and preferably between 9 and 12 and can be assured by using a buffer solution or a solution of an alkaline agent such as sodium hydroxide or potassium hydroxide.

23. Process according to one of Claims 15 to 22, characterized in that the hydrophobic phase immiscible with the abovementioned aqueous phase is produced from hydrophobic liquid substances selected from the group consisting of halogenated or non-halogenated hydrocarbons such as cyclohexane, chloroform or dichloromethane, fatty acid esters such as isopropyl myristate or ethyl oleate, mixtures of fatty acid esters such as, for example, the product Dragoxat®, vegetable oils such as olive oil, sweet-almond oil or groundnut oil, mineral oils such as a paraffin oil, and any mixture of these hydrophobic liquid substances.

24. Process according to one of Claims 15 to 23, characterized in that one or more active substances, in the form of a solution, suspension or emulsion, in particular a preferably insoluble mineral substance which reflects solar radiation, an oil or an oily solution of a liposoluble substance such as a liposoluble sunscreen, are incorporated into the phase to be dispersed, which forms the liquid phase to be encapsulated.

25. Composition, particularly a cosmetic or pharmaceutical composition, especially a dermatological composition, a dietetic composition or a food composition, characterized in that it comprises microcapsules with a wall of crosslinked plant polyphenols, as defined in any one of Claims 1 to 14 or obtained by carrying out the process according to any one of Claims 15 to 24.

26. Composition according to Claim 25, characterized in that the concentration of microcapsules with a wall of crosslinked plant polyphenols is between 0.01 and 10% by weight of the total weight of the final composition and preferably between 0.1 and 5% by weight of the final composition.

27. Composition according to Claim 25 or 26, characterized in that the microcapsules with a wall of crosslinked plant polyphenols take the form of microcapsules in the as-manufactured state, *i.e.* fresh.

28. Composition according to Claim 25 or 26, characterized in that the microcapsules with a wall of crosslinked plant polyphenols are in a dehydrated form, produced especially by lyophilization.

29. Composition according to one of Claims 25 to 28, characterized in that it is intended for preventing ageing of the skin, especially actinic ageing.

30. Cosmetic method of treating a human in order to prevent ageing of the skin, especially the actinic ageing generally due to free radicals, characterized in that an effective amount of microcapsules with a wall of crosslinked plant polyphenols, as defined in any one of Claims 1 to 14, optionally included in a cosmetically or pharmaceutically acceptable excipient, vehicle or carrier, is applied to the areas of the skin or hair which are sensitive to the action of free radicals, especially the free radicals resulting from actinic exposure.

31. Cosmetic method of treatment according to Claim 30, characterized in that the concentration of microcapsules is normally between 0.01 and 10% by weight and preferably between 0.1 and 5% by weight of the composition containing the microcapsules.

32. Process for the preparation of a composition incorporating one or more plant polyphenols, characterized in that, in order to prevent any impairment, especially any colour modification of the composition over time, while at the same time preserving the activity of the plant polyphenols, especially the biological activity, the said polyphenols are incorporated into the said composition in the form of microcapsules as defined according to any one of Claims 1 to 14 or obtained by carrying out the process according to any one of Claims 15 to 24.

33. Process according to Claim 32, characterized in that the composition is selected from the group consisting of a cosmetic or pharmaceutical composition, especially a dermatological composition, a dietetic composition or a food composition.

34. Process according to Claim 32 or 33, characterized in that the activity preserved by the plant polyphenols, in particular by the flavonoids, is an anti-free radical and/or antioxidizing activity.

**Patentansprüche**

1. Mikrokapseln, dadurch charakterisiert, daß sie eine Wand umfassen, die aus einem oder mehreren pflanzlichen vernetzten Polyphenolen gebildet wird, insbesondere mit Hilfe einer Grenzflächen-Vernetzung zwischen dem oder den pflanzlichen Polyphenolen und einem Vernetzungsmittel, bevorzugt einem Disäurehalogenid, insbesondere einem Disäurechlorid.

2. Mikrokapseln nach Anspruch 1, dadurch charakterisiert, daß sie ein Protein oder ein Polysaccharid, oder ein Polyalkylenglykol oder eine beliebige Mischung dieser Substanzen umfassen.

3. Mikrokapseln nach Anspruch 2, dadurch charakterisiert, daß ihre Wand ein Protein und/oder ein Polysaccharid und/oder Polyalkylenglykol umfaßt, das mit dem oder den vorstehend genannten pflanzlichen Polyphenolen covernetzt ist.

4. Mikrokapseln nach einem der Ansprüche 2 oder 3, dadurch charakterisiert, daß das vorstehend angegebene Protein eine spezifische biologische Aktivität aufweist, wie eine enzymatische Aktivität, wie beispielsweise Katalase, Superoxiddismutase oder Glutathionperoxidase, wobei diese Aktivität zu der eigenen Aktivität des oder der pflanzlichen Polyphenole dazukommen kann.

5. Mikrokapseln nach einem der Ansprüche 1 bis 4, dadurch charakterisiert, daß die vorstehend angegebenen pflanzlichen Polyphenole monocyclische oder polycyclische pflanzliche Polyphenole sind wie Flavonoide, Isoplavonoide, Neoflavonoide, Gallotannine, Ellagotannine, Catechin oder seine Derivate wie DL-3,4-Dihydroxyphenylalanin oder DL-DOPA, oder Catecholamine wie 3-Hydroxytyramin oder Dopamin, oder Phloroglucinol, oder Phenolsäuren wie Kaffeesäure, Dihydrokaffeesäure, Protokatechinsäure, Chlorogensäure, Isochlorogensäure, Gentisinsäure, Homogentisinsäure, Gallensäure, Hexahydroxydiphensäure, Ellagsäure, Rosmarinsäure, Lithospermsäure oder die Derivate von Phenolsäuren, insbesondere ihre Ester oder Heteroside, oder Curcumin, oder polyhydroxylierte Cumarine, oder Lignane oder polyhydroxylierte Neolignane oder eine Mischung, die eine oder mehrere pflanzliche Polyphenole oder deren Derivate umfaßt, wie Silymarin, wobei alle vorstehend angegebenen Polyphenole in Form von Präparaten verwendet werden können, die ausgehend von Pflanzen oder Pflanzenteilen wie Extrakten, Tinkturen, Fruchtsäften oder Weinen erhalten werden.

6. Mikrokapseln nach einem der vorstehenden Ansprüche, dadurch charakterisiert, daß die vorstehend angegebenen Flavonoide ausgewählt werden können aus der Gruppe, die aus einem Flavon wie Apigenol, Luteolol, einem Flavonol wie Quercetin, Kämpferol oder einem Flavon- oder Flavonol-Heterosid wie Rutin und seinen Derivaten, einem Flavanon, wie Flavanon, Naringenin, Hesperetin oder einem Flavanon-Heterosid wie Naringin, Hesperidin, Diosmin oder einem Flavanon-Derivat wie Diosmosid oder einem Biflavonoid oder einem Dimer von Flavon oder Flavonon wie Amentoflavon oder einem Chalcon wie Isoliquirtigenin oder Hesperidinmethylchalcon, einem Flavanonol wie Taxifoliol oder einer von Taxifoliol abgeleiteten Substanz wie Silybin, Silychristin, Silydianin, einem Flavan-3-ol wie (+)Catechin, (-)Epicatechin oder einem aus Struktureinheiten auf Flavan-3-ol-Basis gebildeten Polymer, im allgemeinen mit dem Namen "Proanthocyanidin" oder mit dem Begriff "kondensiertes Tannin" bezeichnet, insbesondere einem 2 bis 8 dieser Einheiten umfassenden Oligomeren, im allgemeinen "procyanidolisches Oligomer" (OPC) genannt, einem Anthocyanosid wie Malvosid oder einer ein oder mehrere Flavonoide enthaltende Mischung besteht, insbesondere in Form von Fruchtextrakten oder Pflanzenextrakten oder Pflanzenteilen.

7. Mikrokapseln nach Anspruch 6, dadurch charakterisiert, daß die Flavonoide enthaltenden Mischungen aus diversen Citrus (Rutaceen) extrahierte Mischungen von Citroflavonoiden, eine aus Silybum marianum (Komposeen) extrahierte Mischung von Flavonoiden oder Silymarin, Extrakte von Gingko biloba (Ginkgoaceen), an Anthocyanosiden aus Blaubeeren, Johannisbeeren, Weintraubenschalen, Blättern von rotem Wein reiche Extrakte, Fruchtsäften wie Traubensaft, Johannisbeersaft als solche oder in konzentrierter oder getrockneter Form wie durch Vernebelung oder Lyophilisierung, Rotweine als solche oder konzentriert oder als Trockenmasse oder ihre diversen Mischungen umfassen können.

8. Mikrokapseln nach einem der Ansprüche 2 bis 7, dadurch charakterisiert, daß das vorstehend angegebene Protein ausgewählt werden kann aus der Gruppe, die aus Albuminen wie Serumalbumin, Ovalbumin, alpha-Lactalbumin, den Globulinen, Fibrinogen, Kasein, Pflanzenproteinen wie Sojaproteinen, Glutelinen, die bevorzugt abgebaut sind, solubilisierten Skleroproteinen, Kollagen, Atelokollagen, Gelatine, Hydrolysaten von Gelatine, Peptonen, Hämoglobin, Enzymen wie Katalase, Superoidismutase, Glutathionperoxidase, hydrophile Proteine enthaltenden Mischungen wie Vollmilch oder vollständig oder teilweise entrahmter Milch, Milchpulver, Kondensmilch, Laktoserum-Proteinen, Sojamehl, Atelokollagen-Mischungen und Glykosaminoglykanen besteht.

9. Mikrokapseln nach einem der Ansprüche 2 bis 8, dadurch charakterisiert, daß das vorstehend angegebene Polysaccharid ausgewählt werden kann aus der Gruppe, die aus Dextranen, Alginsäure und ihren wasserlöslichen Salzen, insbesondere Natriumalginat, Pflanzengummis, Carrageenen, Pektinen, löslichen Stärkederivaten, löslichen Cellulosederivaten, Glycosaminoglykanen besteht.

10. Mikrokapseln nach einem der Ansprüche 2 bis 9, dadurch charakterisiert, daß das vorstehend angegebene Polyalkylenglykol ausgewählt werden kann aus der Gruppe, die aus Polyethylenglykolen und Polypropylenglykolen besteht.

11. Mikrokapseln nach einem der vorstehenden Ansprüche, dadurch charakterisiert, daß sie durch Grenzflächen-Vernetzung ausgehend von einer Emulsion hergestellt werden, deren wäßrige Phase 1 bis 40 Gew.-%, bevorzugt 1 bis 20 Gew.-% pflanzliche Polyphenole bezogen auf das Gesamtgewicht der wäßrigen Phase enthält; wenn ein vorstehend genanntes Protein und/oder Polysaccharid und/oder Polyalkylenglykol anwesend ist, ist die Gesamtkonzentration dieser Substanz(en) in der wäßrigen Phase vorteilhafterweise zwischen 0,1 und 30 Gew.-%, bevorzugt zwischen 1 und 10 Gew.-% bezogen auf das Gesamtgewicht der wäßrigen Phase.

12. Mikrokapseln nach einem der vorstehenden Ansprüche, dadurch charakterisiert, daß sie eine oder mehrere wasserlösliche, fettlösliche oder unlösliche Substanzen im Zustand einer Lösung, Suspension oder Emulsion enthalten, insbesondere eine Sonnenstrahlen reflektierende mineralische Substanz, bevorzugt unlöslich, ein Pflanzenöl oder eine ölige Lösung, die eine fettlösliche aktive Substanz enthält wie einen fettlöslichen Sonnenfilter.

13. Mikrokapseln nach Anspruch 12, dadurch charakterisiert, daß die vorstehend angegebene aktive Substanz, die in den Mikrokapseln vorliegt, aus der Gruppe ausgewählt wird, die aus einer Sonnenstrahlen reflektierenden mineralischen Substanz wie einem Eisenoxid, Titanoxid, Zinkoxid, Talkum, Kaolin, einem Pflanzenöl wie einem Getreidekeimöl oder einem desodorierten Fischleberöl oder einer öligen Lösung einer fettlöslichen Substanz wie Vitamin A, Vitamin D2, Vitamin E oder Tocopherol, einer essentiellen Fettsäure wie Linolsäure, Linolensäure, Arachindonsäure, einem Ceramid, einem fettlöslichen Derivat von Ascorbinsäure wie Ascorbylpalmitat oder einem fettlöslichen Sonnenfilter wie einem Zimtsäureester, einem Paraaminobenzoesäureester, einem Salicylester, einem Benzophenon, Benzylidencampher und seinen Derivaten, ein Derivat von Dibenzoylmethan, einem Benzimidazol oder einer photoaktiven Substanz wie Bergapten oder jedem anderen Derivat von Psoralen oder einer mehrere aktive Substanzen enthaltenden Mischung besteht.

14. Mikrokapseln nach einem der vorstehenden Ansprüche, dadurch charakterisiert, daß sie einen Durchmesser zwischen 0,1 $\mu$m (Mikrometer) und 3 mm zeigen.

15. Verfahren zur Herstellung von Mikrokapseln, dadurch charakterisiert, daß man eine Grenzflächen-Vernetzung einer Emulsion vom Typ Wasser-in-Öl durchführt, umfassend die wesentlichen folgenden Schritte:

a) man stellt eine wäßrige Phase her, die das zu vernetzende Pflanzenpolyphenol oder die Mischung der Pflanzenpolyphenole enthält,

b) man stellt eine hydrophobe Phase her, die gegebenenfalls einen oder mehrere oberflächenaktive Stoffe enthält,

c) man emulgiert die wäßrige Phase in der vorstehend angegebenen hydrophoben Phase, so daß die hydrophobe Phase die kontinuierliche Phase bildet, in der die wäßrige Phase die dispergierte Phase bildet,

d) man gibt zur so erhaltenen Emulsion unter Rühren ein in einer mit der hydrophoben Phase mischbaren Flüssig gelöstes Vernetzungsmittel, um eine Grenzflächen-Vernetzung des Vernetzungsmittels und des oder der Pflanzenpolyphenole zu bewirken, die in der wäßrigen Phase enthalten sind,

e) man rührt während einer Reaktionszeit weiter, die zur Erzielung einer ausreichenden Vernetzung geeignet ist, welche zur Bildung von Mikrokapseln führt, deren Wand das oder die durch das Vernetzungsmittel vernetzten Pflanzenpolyphenole umfaßt,

f) man isoliert die so gebildeten Mikrokapseln mit jedem geeigneten Mittel.

16. Verfahren zur Herstellung von Mikrokapseln, dadurch charakterisiert, daß man eine Grenzflächen-Vernetzung einer Emulsion vom Typ Öl-in-Wasser durchführt, umfassend die folgenden wesentlichen Schritte:

a) man stellt eine hydrophobe Phase her, in der man das Vernetzungsmittel löst,

b) man stellt eine wäßrige Phase her, die das zu vernetzende Pflanzenpolyphenol oder die Mischung der Pflanzenpolyphenole und gegebenenfalls ein oder mehrere oberflächenaktive Stoffe enthält,

c) man emulgiert die hydrophobe Phase in der vorstehend angegebenen wäßrigen Phase, so daß die wäßrige Phase die kontinuierliche Phase bildet, in der die hydrophobe Phase die dispergierte Phase bildet,

d) man rührt während einer Reaktionszeit weiter, die zur Erzielung einer ausreichenden Vernetzung geeignet ist, welche zur Bildung von Mikrokapseln führt, deren Wand das oder die durch das Vernetzungsmittel vernetzten Pflanzenpolyphenole umfaßt,

e) man isoliert die so gebildeten Mikrokapseln mit jedem geeigneten Mittel.

17. Verfahren nach Anspruch 15 oder dadurch charakterisiert, daß man zur wäßrigen Phase während ihrer Herstellung ein Protein oder ein Polysaccharid oder ein Polyalkylenglykol oder eine beliebige Mischung dieser Substanzen zugibt.

18. Verfahren nach einem der Ansprüche 15 bis 17, dadurch charakterisiert, daß man die gebildeten Mikrokapseln zur Entfernung des überschüssigen Vernetzungsmittels genauso wie unreagierter oder nicht in den Mikrokapseln eingekapselter Pflanzenpolyphenol(e) wäscht.

19. Verfahren nach einem der Ansprüche 15 bis 18, dadurch charakterisiert, daß die Mikrokapseln lyophilisiert sind.

20. Verfahren nach einem der Ansprüche 15 bis 19, dadurch charakterisiert, daß das Vernetzungsmittel ein Disäurehalogenid, insbesondere ein Disäurechlorid umfaßt, bevorzugt ausgewählt aus der Gruppe, die aus einem Chlorid einer aliphatischen oder aromatischen Disäure besteht wie Sebacoylchlorid, Succinylchlorid, Adipoylchlorid, Terephthaloylchlorid, Glutarylchlorid.

21. Verfahren nach einem der Ansprüche 15 bis 20, dadurch charakterisiert, daß die Konzentration an Disäurehalogenid zwischen 0,2 und 10 Gew.-% liegt, bezogen auf das Gesamtgewicht des Reaktionsmediums.

22. Verfahren nach einem der Ansprüche 15 bis 21, dadurch charakterisiert, daß der pH der Reaktion zwischen 8 und 14, besser zwischen 9 und 12 liegt und vielleicht durch eine Pufferlösung oder eine Lösung eines alkalischen Mittels wie Soda oder Pottasche gewährleistet wird.

23. Verfahren nach einem der Ansprüche 15 bis 22, dadurch charakterisiert, daß die nicht mit der vorstehend angegebenen wäßrigen Phase mischbare hydrophobe Phase ausgehend von hydrophoben flüssigen Substanzen hergestellt wird, die ausgewählt werden aus der Gruppe, welche aus halogenierten oder nicht halogenierten Kohlenwasserstoffen wie Cyclohexan, Chloroform oder Dichlormethan, Fettsäureestern, wie Isopropylmyristat oder Ethyloleat, Mischungen von Fettsäureestern wie beispielsweise dem Produkt Dragoxat$^{®}$, Pflanzenölen wie Olivenöl, Mandelöl oder Erdnußöl, Mineralölen wie einem Paraffinöl und jeder Mischung dieser hydrophoben flüssigen Substanzen besteht.

24. Verfahren nach einem der Ansprüche 15 bis 23, dadurch charakterisiert, daß man der zur dispergierenden Phase, die die einzukapselnde flüssige Phase bildet, eine oder mehrere aktive Substanzen im Zustand einer Lösung, Suspension oder Emulsion zusetzt, insbesondere eine Sonnenstrahlen reflektierende mineralische Substanz, bevorzugt unlöslich, ein Öl oder eine ölige Lösung einer fettlöslichen Substanz wie eines fettlöslichen Sonnenfilters.

25. Zusammensetzung, insbesondere kosmetische oder pharmazeutische, insbesondere dermatologische Zusammensetzung, diätetische Zusammensetzung oder Lebensmittel-Zusammensetzung, dadurch charakterisiert, daß sie Mikrokapseln mit einer Wand aus vernetzen Pflanzenpolyphenolen umfaßt, wie in einem der Ansprüche 1 bis 14 definiert, oder erhalten durch Durchführung des Verfahrens nach einem der Ansprüche 15 bis 24.

26. Zusammensetzung nach Anspruch 25, dadurch charakterisiert, daß die Konzentration der Mikrokapseln mit einer Wand aus vernetzten Pflanzenpolyphenolen zwischen 0,01 und 10 Gew.-% des Gesamtgewichts der Endzusammensetzung liegt, besser zwischen 0,1 und 5 Gew.-% der Endzusammensetzung.

27. Verfahren nach Anspruch 25 oder 26, dadurch charakterisiert, daß die Mikrokapseln mit einer Wand aus vernetzten Pflanzenpolyphenolen in Form von roh hergestellten Mikrokapseln vorliegen, die frisch genannt werden.

28. Zusammensetzung nach Anspruch 25 oder 26, dadurch charakterisiert, daß die Mikrokapseln mit einer Wand aus vernetzten Pflanzenpolyphenolen in getrockneter Form vorliegen, insbesondere durch Lyophilisierung.

29. Zusammensetzung nach einem der Ansprüche 25 bis 28, dadurch charakterisiert, daß sie zur Verhinderung der Hautalterung bestimmt ist, insbesondere der aktinischen Alterung.

30. Verfahren zur kosmetischen Behandlung eines Menschen zur Verhinderung der Hautalterung, insbesondere der aktinischen Alterung, die im allgemeinen auf freie Radikale zurückzuführen ist, dadurch charakterisiert, daß man eine wirksame Menge von Mikrokapseln mit einer Wand aus vernetzten Pflanzenpolyphenolen wie in einem der Ansprüche 1 bis 14 definiert, gegebenenfalls in einem kosmetisch oder pharmazeutisch verträglichen Excipienten, Vehikel oder Träger eingeschlossen, auf die Bereiche der Haut oder der Haare anwendet, die für die Wirkung von freien Radikalen empfindlich sind, insbesondere von freien Radikalen, die aus einer aktinischen Belichtung resultieren.

31. Verfahren zur kosmetischen Behandlung nach Anspruch 30, dadurch charakterisiert, daß die Konzentration an Mikrokapseln üblicherweise zwischen 0,01 und 10 Gew.-%, bevorzugt zwischen 0,1 und 5 Gew.-% der die Mikrokapseln enthaltenden Zusammensetzung liegt.

32. Verfahren zur Herstellung einer ein oder mehrere Pflanzenpolyphenole beinhaltenden Zusammensetzung, dadurch charakterisiert, daß man im Hinblick auf die Verhinderung jeder Veränderung, insbesondere jeder Änderung der Farbe der Zusammensetzung im Verlauf der Zeit, wobei die Aktivität der Pflanzenpolyphenole, insbesondere die biologische Aktivität aufrechterhalten wird, diese in die Zusammensetzung in Form von Mikrokapseln eingearbeitet werden, die wie in einem der Ansprüche 1 bis 14 definiert sind oder durch Durchführung des Verfahrens nach einem der Ansprüche 15 bis 24 erhalten werden.

33. Verfahren nach Anspruch 32, dadurch charakterisiert, daß die Zusammensetzung ausgewählt wird aus der Gruppe, die aus einer kosmetischen oder pharmazeutischen, insbesondere dermatologischen Zusammensetzung, einer diätetischen Zusammensetzung oder einer Lebensmittel-Zusammensetzung besteht.

34. Verfahren nach Anspruch 32 oder 33, dadurch charakterisiert, daß die durch die Pflanzenpolyphenole, insbesondere die Flavonoide aufrechterhaltene Aktivität eine antiradikalische und/oder antioxidierende Wirkung ist.